# EUROPEAN PATENT SPECIFICATION

(11) **EP 1 214 598 B1**
(45) Date of publication and mention of the grant of the patent: **17.05.2006**
(21) Application number: 00965965.7
(22) Date of filing: 11.09.2000
(51) Int. Cl.: G01N 33/68, C07K 14/47, C07K 16/18

(54) **SCREENING FOR INHIBITORS OF "PAIRED HELICAL FILAMENTS"**
SCREENING NACH INHIBITOREN VON "GEPAARTEN HELIKALEN FILAMENTEN"
CRIBLAGE D'INHIBITEURS DE "FILAMENTS HELICOIDAUX APPARIES"

(30) Priority: 09.09.1999 EP 99117805
(43) Date of publication of application: 19.06.2002
(73) Proprietor: Max-Planck-Gesellschaft zur Förderung der Wissenschaften e.V., Berlin (DE)
(72) Inventor: VON BERGEN, Martin, 21483 Gülzow (DE); BIERNAT, Jacek, 22869 Schenefeld (DE); MANDELKOW, Eva-Maria, 22607 Hamburg (DE); MANDELKOW, Eckhard, 22607 Hamburg (DE)
(74) Representative: Vossius & Partner
(86) International application number: PCT/EP2000/008863
(87) International publication number: WO 2001/018546

(56) References cited:
- WO-A-96/30766
- WO-A-99/62548
- VON BERGEN M ET AL: "Rapid assembly of microtubule-associated protein tau into Alzheimer-like paired helical filaments monitored by fluorescence in solution." MOLECULAR BIOLOGY OF THE CELL, vol. 9, no. SUPPL., November 1998 (1998-11), page 394A XP000986927 38th Annual Meeting of the American Society for Cell Biology;San Francisco, California, USA; December 12-16, 1998 ISSN: 1059-1524
- VON BERGEN M ET AL: "Assembly of tau protein into Alzheimer paired helical filaments depends on a local sequence motif (306VQIVYK311) forming beta structure." PROCEEDINGS OF THE NATIONAL ACADEMY OF SCIENCES OF THE UNITED STATES, vol. 97, no. 10, 9 May 2000 (2000-05-09), pages 5129-5134, XP000982226 May 9, 2000 ISSN: 0027-8424

## Description

The present invention relates to a method for identifying and obtaining an inhibitor, capable of modifying the PHF ("paired helical filaments") formation comprising the steps of (a) incubating a peptide consisting of a specific tau derived peptide as defined herein with a compound to be screened under conditions which permit assembly of said tau-derived peptides into nucleation sites for PHF assembly and/or into aggregation products; and (b) detecting the presence, decrease, or absence of nucleation sites for PHF assembly and/or the presence, decrease or absence of said aggregation products wherein said absence and/or decrease is indicative for putative inhibitors for PHF formation. Furthermore, the present invention provides inhibitors identified or obtained by said method as well as compositions comprising said inhibitor, wherein said composition is preferably a diagnostic and/or a pharmaceutical composition. The present invention further relates to a method for detecting and/or measuring PHF formation comprising the steps of (a) incubating a peptide consisting of a specific tau derived peptide as defined herein with tau-proteins and/or fragments thereof under conditions which permit assembly of tau-proteins and/or fragments thereof into PHFs; and (b) detecting the presence, absence, decrease or increase of PHFs and/or nucleation sites of PHF assembly.

Alzheimer's disease and other dementias are characterized by abnormal protein deposits in the brain, such as amyloid plaques or neurofibrillary tangles, formed by fibrous assemblies of the Aβ peptide or of tau protein, wherein these tangles comprise tau protein aggregated into so-called paired helical filaments (PHFs) as reviewed in Lee and Trojanowski (1992), Curr. Opin. Neurobiol. 2, 653-656. These aggregates are thought to be toxic to neurons, either by causing some toxic signaling defect (in the case of Aβ) or by obstructing the cell interior (in the case of tau deposits). It is therefore one of the top priorities in Alzheimer research to understand the reasons for the pathological aggregation in order to find methods to prevent it. The principles governing Aβ aggregation are understood in some detail: The peptide (40-43 residues) is a short derivative of a much larger membrane protein (APP) which is cleaved in an inappropriate fashion (by β and γ secretases); the peptide is largely hydrophobic because it contains the membrane insertion sequence which tends to promote aggregation, and it undergoes a conformational change to a β-conformation which allows fiber assembly with a "cross β" interaction (Barrow, J. Mol. Biol. 225 (1992), 1075-1093; Hilbich, J. Mol. Biol. 228 (1992), 460-473; Jarrett and Lansbury, Cell 74 (1993), 1055-1058; Kirschner, Proc. Natl. Acad. Sci. USA 83 (1986), 503-507). A similar cross-β structure is found in other disease-related proteins, such as fibrous prion protein (in Creutzfeld-Jacob disease) or transthyretin fibers (in systemic amyloidosis, reviews Castano and Frangione, Curr. Opin. Neurology 8 (1995), 279-285; Lansbury, Proc. Natl. Acad. Sci. USA 96 (1999), 3342-3344).

By contrast, the principles governing tau aggregation have remained elusive (review, Friedhoff & Mandelkow, 1999). Tau is not only soluble and displays a 'natively unfolded' structure (as mentioned, inter alia, in Mandelkow (1998), TICB 8, 425-427), but is one of the most hydrophilic proteins known, a fact that strongly argues against hydrophobic interactions. Tau can aggregate as an intact protein, 352-441 residues in length (depending on isoform), so that all 6 tau isoforms are found in Alzheimer paired helical filaments (PHFs) (Goedert, Neuron 8 (1992), 159-168). PHFs are therefore made up of tau protein, forming the neurofibrillary tangles. In tau, there are either 0, 1, or 2 inserts of 29 residues each near the N-terminus, and 3 or 4 homologous stretches of 31 residues each, the "repeats" in the C-terminal half (repeat R2 may be missing). Thus, the longest isoform has four repeats and two inserts, the shortest (fetal) isoform has 3 repeats and no inserts. The 4 repeats are followed by a poorly conserved "fifth" repeat (R'). A "big tau" isoform containing -300 additional residues (exon 4a) is expressed in peripheral nerves (Couchie, Proc. Natl. Acad. Sci. USA 89 (1992), 4378-4381). Tau contains either one or two cysteines, residue 322 in repeat 3 (always present), and residue 291 in repeat 2 (present only in 4-repeat isoforms). This difference has an influence on PHF assembly. As mentioned herein above, the amino acid composition of tau is dominated by hydrophilic and charged residues, an acidic stretch near the N-terminus followed by mostly basic domains. The repeats are flanked by regions rich in prolines. The C-terminal half of tau (repeats plus flanking regions) constitutes the microtubule-binding domain. The core of the above-mentioned PHFs (paired ,helical filaments) are mainly built from the repeat domain (Ksiezak-Reding and Yen, Neuron 6 (1991), 717-728; Novak, EMBO J. 12 (1993), 365-370; Wischik, Proc. Natl. Acad. Sci. USA (1988), 4506-4510), and this domain also promotes PHF assembly in vitro (Wille, J. Cell Biol. 118 (1992), 573-584). Tau contains almost no secondary structure but rather appears as a Gaussian random coil, as judged by spectroscopic and X-ray evidence (Cleveland, J. Mol. Biol. 116 (1977), 227-247). These data thus provide no basis for making assumptions about secondary structure interactions. Additionally, as e.g. shown in Schweers (1994), JBC 269, 24290-24297, tau protein behaves in solution as if it were denatured and no evidence for compact folding was detected. Schweers and colleagues employed a variety of spectroscopy methods in order to elucidate the conformation of tau protein and PHFs and came to the conclusion that it is unlikely that the aggregation of tau into Alzheimer PHFs is based on interactions between strands of β-sheets (a model currently favored for other disease-related polymers such as β-amyloid fibers of Alzheimer's disease). Therefore, the nature of the PHF (and/or tau) aggregation mechanism could not simply be modeled on the basis of known protein structures. On the other hand, despite its random coil appearance in solution, tau assembles into a well-defined periodic fiber, the paired helical filament (PHF). This process can be enhanced by oxidation (Wille (1992), loc. cit.), by polyanions (Friedhoff, Biochemistry 37 (1998a), 10223-10230; Goedert, Nature 383 (1996), 550-553; Kampers, FEBS Letters 399 (1996), 344-349; Perez, J. Neurochem. 67 (1996), 1183-1190) and can be described by a nucleation-condensation mechanism (Friedhoff, Proc. Natl. Acad. Sci. USA 95 (1998b), 15712-15717). Here, too, the secondary structure content is below detectability (Schweers, J. Biol. Chem. 269 (1994), 24290-24297). A fraction of tau polymers in Alzheimer brains occurs as straight (untwisted) fibers (Crowther, Proc. Natl. Acad. Sci. USA (1991), 2288-2292), and fibers without axial periodicity have also been described for some in vitro assembly conditions (Montejo de Garcini and Avila, J. Biochem. 102 (1987); 1415-1421; Perez, loc. cit.; Wilson and Binder, Amer. J. Pathol. 150 (1997), 2181-2195).

Because of the long-range periodicity of most PHFs it can be speculated that they are built from a reproducible secondary structure element in the microtubule-binding domain of tau which is responsible for the repetitive and specific interaction that leads to filament aggregation. However, said secondary structure was not yet elucidated and this secondary structure element might be too short to be detected by global spectroscopic studies of intact tau. As mentioned herein above, tau was illustrated as a "Gaussian" polymer and no unique folding was deduced so that many different structures are possible. The relevant structural element might be too short to be detected reliably by global spectroscopic studies of intact tau.

Additionally, PHFs form much more easily from tau constructs containing only the repeat region than from whole tau. Schweers showed in 1995 (Proc. Natl. Acad. Sci. USA 92, 8463-8467) that the repeats are necessary for assembling PHFs in vitro. A SH group in repeat 3 was described to be available for forming intermolecular disulfide linkages and this initial dimerization was considered as the basis for further PHF assembly.
There is a widespread feeling in the art that in general pathologic fibers tend to be "amyloidogenic" in nature, meaning that they consist of proteins with predominant β-structure which leads to the irreversible aggregation. However, in the case of tau, Mandelkow (Neurobiology of Aging 16, 347-354) mentions that in spectrometric experiments no CD-spectrum expected for a protein with β-conformation could be detected when several tau isoforms or constructs derived from them were evaluated. Therefore, it was concluded that fiber aggregation on the basis of β-strand interaction is unlikely in the case of tau, in contrast to the aggregation of β-amyloid.

All previous art, therefore, showed that the repeat region of tau might be involved in PHF nucleation formation and/or aggregation, however, a distinct structural feature of tau which might be responsible for said PHF nucleation formation and/or aggregation was not described. Additionally, genetically engineered constructs were employed in in vitro assays to show that PHFs can arise from a nucleated assembly mechanism which is dependent on the dimerization of tau (Friedhoff, 1998, loc. cit.). However, said in vitro assays provided no insight in the minimal requirements for nucleation, formation of PHFs and/or aggregation of tau protein or tau peptides. Furthermore, test assays and systems for PHF assembly, as described in the prior art and based on full-length tau (see, inter alia, Wille (1992), loc. cit.) or on fragments thereof, comprising major parts of the repeat-domain of tau (e.g. the peptide K19 as described in the appended examples or, inter alia, in Friedhoff (1998), loc. cit.) or a protease-resistant core structure (see, WO96/30766) need incubation times for PHF assembly which vary from several minutes up to several days. Von Bergen (1998), Mol. Biol. Cell 9, Suppl., page 394A describes a spectroscopic assay to monitor PHF-formation, whereby tau was C- and V-terminally truncated. These prior art assays involve the addition of polyanions, like heparin, poly-glutamic acid or RNA and oxidation of SH-groups on the tau protein or the corresponding fragments.
Considering the fact that tau aggregation is much less understood than that of, inter alia, β-amyloid, and even counter-intuitive since tau is cytosolic and one of the most highly soluble proteins known in the art, it is not clear why tau should aggregate in a specific manner to form Alzheimer related PHFs. Furthermore, it is unknown which specific structural principle could be responsible for PHF formation and/or aggregation. Additionally, prior test systems and methods, based on full-length tau or fragments thereof comprising the repeat domain are rather slow and are not capable of providing for fast and reliable assays for PHF formation and/or aggregation of tau or tau derived peptides. Said desired fast and reliable test assays may be used, inter alia, for the testing of potential drugs which may interfer with the above described pathological tau assembly. Accordingly, means and methods for diagnosing and treating Alzheimer's disease on the basis of said structural principle of tau, e.g. with chemotherapeutic treatment of said disease which influences tau aggregation, were hitherto not available but are nevertheless highly desirable.

Thus, the technical problem of the present invention is to comply with the needs described above. The solution to this technical problem is achieved by providing the embodiments characterized in the claims.

Accordingly, the invention relates to a method for identifying and obtaining an inhibitor, capable of modifying the PHF formation comprising the steps of
(a) incubating a peptide consisting of a specific tau derived peptide as shown in any one of SEQ ID NOs 6, 7, 8 and 9, with a compound to be screened under conditions which permit assembly of said tau derived peptides into nucleation sites for PHF assembly and/or into aggregation products; and
(b) detecting the presence, decrease, or absence of nucleation sites for PHF assembly and/or the presence, decrease or absence of said aggregation products wherein said absence and/or decrease is indicative for putative inhibitors for PHF formation.

The term "PHF formation" as used in accordance with this invention means the assembly of tau protein or fragments thereof into paired helical filaments, wherein said paired helical filaments do not only comprise bona fide PHFs but also thin filaments of tau protein or fragments thereof which may serve as nuclei and/or nucleation sites which can efficiently promote the assembly of bona fide PHFs from tau fragments, tau constructs and/or intact tau. For example, the aggregation of smaller tau derived peptides (like PHF43, SEQ ID NO: 6) as described herein (see appended examples) into thin filamentous structures or nucleation sites for PHFs is also considered as "PHF formation". Furthermore, the term "PHF formation" comprises also PHF aggregations and aggregation of peptides comprising tau-fragments. The aggregation of said peptides comprising a specific tau derived peptide as defined herein as well as the dimerization of said peptide is also comprised in said term. Similarly, the term "aggregation products" comprises any aggregation, interaction, assembly and/or dimerization of said peptides. Said "aggregation products" may be measured during their formation and/or as final reaction product. Methods for the measurement of PHF formation and/or the formation of aggregation products are well known in the art and described, inter alia, in the appended examples.
The term "modifying the PHF formation" as used herein is not limited to (complete) "inhibiting" but also comprises "delaying" the PHF formation under suitable in vitro conditions. Such suitable in vitro conditions are known in the art and described, inter alia in Schweers, Proc. Natl. Acad. Sci. USA 92 (1995), 8463-8467, in Friedhoff, Biochemistry 37 (1995), 10223-10230 or in the appended examples. The term furthermore comprises the alteration which may occur during PHF-nucleation, PHF-elongation or even during first dimerization processes which may lead to a nucleation site for PHF formation. A "modified PHF formation" may be measured, inter alia, by testing said PHF-formation in an assay comprising the compound to be screened and in a parallel assay wherein, under the same in vitro conditions, said compound is omitted and/or replaced by an irrelevant compound, like, lysozyme, BSA, tubulin, hemoglobin, etc.

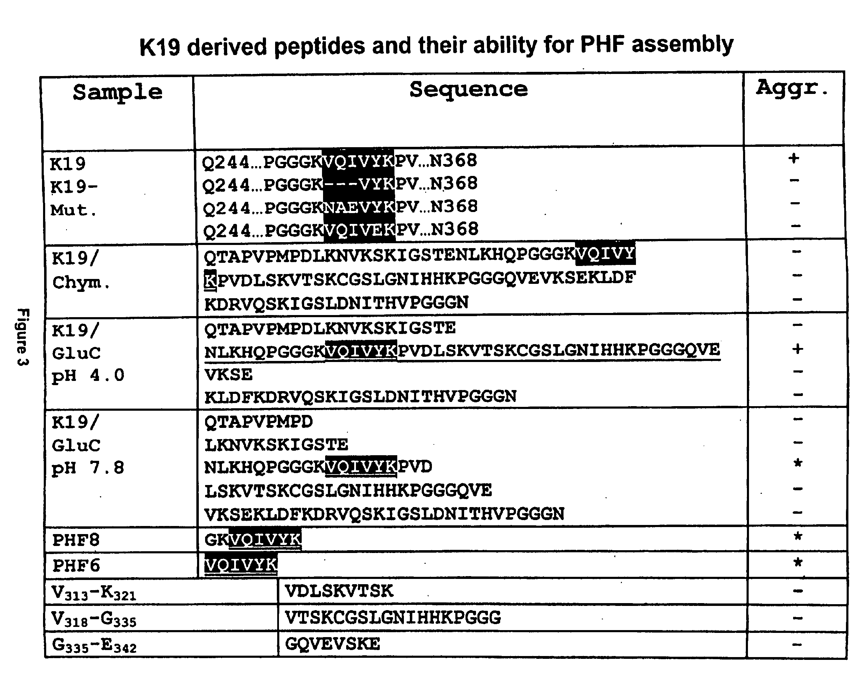

The term "a peptide consisting of a specific tau derived peptide as defined in any one of SEQ ID NOs" means in accordance with the present invention any peptide consisting of the specific amino acid sequence as depicted in the corresponding SEQ ID NOs. Preferably, said sequence(s) is (are) derived from the repeat region of tau. Most preferably, said sequences are derived from the sequence stretch between amino acid 244 to 368 (comprising essentially the whole repeat region of the tau isoforms; numbering of human fetal tau). Particularly preferred, in this context, is a tau-derived peptide fragment consisting of only 43 residues, i.e. essentially the third repeat plus flanking stretches on either site (human fetal tau sequence 265 to 338 without the second repeat 275-305). Most particularly preferred said peptide consisting of the hexapeptide "PHF6", derived from the human fetal tau sequence 306 to 311 (SEQ ID NO: 8), or the hexapeptide "PHF6*", derived from the human fetal tau sequence 275 to 280 (SEQ ID NO: 7). Another peptide consisting of a specific tau derived peptide as defined herein above is, inter alia, shown in SEQ ID NO: 9 and denoted as "PHF8". As defined in the appended examples, said peptide comprises the amino acid sequence as depicted in SEQ ID NO: 8 and two additional amino acids (G and K) derived from the neighbouring amino acid sequence of SEQ ID NO: 8 in the full-length human tau sequence. The (poly)peptides, therefore, consist entirely of the peptides as defined in the corresponding SEQ ID NOs 6, 7, 8 and 9. Preferably, said amino acid chains consist of 6 amino acids, more preferably sais amino acid chain consists of 8 amino acids. Accordingly, particularly preferred is an amino acid chain consisting of 6, 8 or 43 amino acids. In a particularly preferred embodiment said amino acid chain is the amino acid sequence as represented in SEQ ID NO: 6 comprising the amino acid sequence as represented in SEQ ID NO: 8 as well as amino acid sequences derived from tau.
Within the scope of the present invention are also peptides consisting of tau derived peptides which are homologous to the above described peptides as depicted in SEQ ID NOs: 6 to 9, but are derived from different species than human. Especially preferred tau homologues are in this context tau-derived peptides from mouse, rat, cow, sheep, polar bear, etc. Furthermore, the present invention also relates to methods, compositions, uses and kits wherein said peptide comprising (a) tau derived peptide(s) is a peptide which is similar to the above described peptides, but wherein said amino acids V, Q and/or I (Valine, Glutamine, Isoleucine) have been replaced, either alone or in combination, by other amino acids which show high intrinsic β-sheet propensities. These amino acids comprise, inter alia T, F, Y (Threonine, Phenylalanine, Tyrosine). Amino acids having a high intrinsic β-sheet propensity are well known in the art and described, inter alia, in Street, (1999), P.N.A.S. 96, 9074-9076. Amino acids which do not have an intrinsic β-sheet propensity, like Proline (P), are also known in the art and can, accordingly, not replace any of the amino acids as defined in the hexapeptide as disclosed herein and depicted in SEQ ID NO: 7 or 8 in order to obtain an assembly competent minimal tau protein/peptide. Therefore, within the scope of the present invention, are also methods and uses wherein functional homologues of the amino acid molecules as depicted in SEQ ID NOS. 6 to 9 are employed. Said functional homologues may comprise, inter alia, the above-metnioned amino acids T, F or Y or other amino acids having a high intrinsic β-sheet propensity. Yet, it is furtermore envisaged that the the above mentioned amino acids, which do not have intrinsic β-sheet propensity, may be employed in specific tests and methods. It is, e.g. envisaged that one or more amino acids having high intrinsic β-sheet propensity be replaced in the hexapeptide structure as disclosed in SEQ ID NOS 7 or 8 and that said replacement(s) lead(s) to minimal-tau peptides with limited or no β-structure formation capacities. Such "mutated" minimal-tau peptides are particulary useful in animal and/or tissue or cell culture systems or in vitro systems as described herein. It is, e.g., envisaged that in the hexapeptides as described herein, the V, Q and/or I is replaced by N, A and/or E (see also appended examples and Fig. 3) or that (a) single residue(s) in the hexapeptide stretch is/are replaced by proline(s). It is understood that such a replacement may be performed in any minimal-tau peptide as disclosed herein; i.e. minimal-tau peptides as depicted in any of SEQ ID NOS 6 to 9.
All methods, uses and assays described herein are not only useful for scientific purposes but are in particular useful in drug screening methods and for pharmaceutical/pharmacological tests and assays.
As mentioned herein below, it is not only preferred that animal-, tissue- or cell culture systems be employed, but also in vitro test/assay systems are within the scope of the present invention. In vivo and in vitro systems are well known in the art and comprise, inter alia, the production and use of transgenic animals expressing a minimal-tau peptide as defined herein (either mutated or non-mutated), as well as corresponding tissue- and/or cell culture systems. Yet, it is particulary preferred that the minimal tau proteins/peptides as defined herein (either mutated or non-mutated) be employed in in vitro systems which also comprise high-throughput screens.

The peptides/(poly)peptides may be produced recombinantly using a polynucleotide sequence that encodes tau and/or a fragment thereof or may be produced by biochemical or synthetic techniques. Those methods are known to those of ordinary skill in the art (see, e.g. Sambrook et al., Molecular Cloning: A Laboratory Manual, Cold Spring Harbor Laboratory Press, Cold Spring Harbor, New York, 1989; Merrifield, J. Am. Chem. Soc. 85 (1963), 2149-2146; Stewart, "Solid Phase Peptide Synthesis", WH Freeman Co, San Francisco (1969); Scopes, "Protein Purification", Springer Verlag, New York, Heidelberg, Berlin (1987); Janson, "Protein Purification, Principles, High Resolution Methods and Applications", VCH Publishers, New York, Weinheim, Cambridge (1989); Wrede, "Concepts in Protein Engineering and Design", Walter de Gruyter, Berlin, New York (1994)).

Furthermore, it is envisaged that nucleic acid molecules encoding the minimal tau peptide as defined herein be employed in the method of the present invention. It is, inter alia, envisaged that the minimal tau peptide be expressed in corresponding test systems which comprise animals, preferably transgenic animals, and/or cell or tissue culture systems as described herein below. Preferably, said transgenic animal and/or cell or tisue culture system expresses an amino acid molecule as shown in any one of SEQ ID NOS. 6 to 9.

Additionally, within the scope of the invention are peptides wherein the above mentioned amino acid(s) and/or peptide bonds have been replaced by functional analogs, inter alia by peptidomimetics. Therefore, the present invention also encompasses functional derivatives and/or analogues of said peptides comprising a specific tau-derived peptide which is capable of self-assembly and/or the formation of nucleation sites for PHF-formation. Particularly preferred are peptides comprising specific tau derived peptides which are capable of a β-sheet like interaction. Methods for the preparation of said peptides, (chemical) derivatives and/or analogues are well known to those in the art and comprise, inter alia, recombinant preparation (as described, inter alia, in Biernat (1992), EMBO J. 11, 1593-1597), protein/peptide isolation and purification (as described, inter alia, in Schweers (1995), loc. cit.), or proteolytic methods as described, inter alia, in the appended examples. Further methods for the preparation of peptides/(poly)peptides are described in Sambrook et al., loc. cit., or in Oxender and Fox (1987), "Protein preparation of chemical derivates and/or analogues are described in, for example, Beilstein "Handbook of Organic Chemistry", Springer Edition New York, or in "Organic Synthesis", Wiley, New York.
Furthermore, the term "peptide comprising a specific tau derived peptide as defined in any one of SEQ ID NOs" also relates to a fusion protein/peptide comprising a peptide as defined herein above. Therefore, the "peptide comprising a specific tau derived peptide as defined in any one of SEQ ID NOs" can comprise (a) further domain(s), said domain(s) being linked by covalent or non-covalent bonds. The linkage can be based on genetic fusion according to the methods known in the art (Sambrook et al., loc. cit., Ausubel, loc. cit.) or can be performed by, e.g., chemical cross-linking as described in, e.g. WO 94/04686. The additional domain present in the fusion protein/peptide comprising the above mentioned peptide comprising a specific tau derived peptide may be joined directly (i.e. with no intervening amino acids) or may be linked by a flexible linker, advantageously a polypeptide linker, wherein said polypeptide linker comprises plural, hydrophilic, peptide-bonded amino acids of a length sufficient to span the distance between the C-terminal end of said further domain and the N-terminal end of the peptide or vice versa. Said polypeptide linker might be a cleavable linker, e.g. a linker cleavable by specific enzymes.
Said fusion protein/peptide may comprise other functional amino acid sequences, like protein tags (GST, GFP, h-myc-peptide, FLAG, etc.) which are derived from heterologous proteins. These further protein tags may be used, inter alia, in adopted detection systems as described herein in the appended examples.

The term "nucleation sites for PHF assembly" as used in accordance with the present invention means the complex or aggregate formation between at least two peptides consisting of a specific tau derived peptide as defined in any one of SEQ IDs 6, 7, 8, or 9. The term furthermore comprises short or thin filaments formed in vitro without a paired helical appearance but a high competence to nucleate bona fide PHFs from full length tau and/or fragments thereof. Therefore, the term "nucleation sites for PHF assembly" comprises short dimers (homodimers or heterodimers) of peptides consisting of said tau derived peptides or fragments thereof as well as "nuclei" of higher structure which can then elongate into PHFs. The detection of the presence, decrease or absence of such nucleation sites for PHF assembly is well known in the art as described in Friedhoff (1998a, b) loc. cit. or is illustrated in the appended examples. The detection can be carried out, inter alia, by microscopic and/or spectroscopic methods as explained herein below. Further particular preferred detection methods comprise fluorescence spectroscopy, especially fluorescence spectroscopy employing thioflavine S or T or the detection of the self-fluorescence of tyrosine (like Tyr310 of the human tau sequence, see, inter alia PHF6 or PHF8 as depicted in SEQ ID NOS: 7 and 8, or of PHF43 or K19 fragments as depicted in SEQ ID NO: 6 or 57), as described in the appended examples. Said detection of tyrosine self-fluorescence is particularly preferred when short tau-derived peptides and their aggregation, assembly and/or dimerization is measured.

In accordance with this invention, potential inhibitors, capable of modifying the PHF formation may be (an) antibody(ies). Said antibody(ies) may comprise (a) monoclonal antibody/antibodies, (a) polyclonal antibody(ies) or (a) chimeric antibody(ies). Furthermore, said antibody(ies) comprise(s) synthetic antibodies, antibody fragments, or a chemically modified derivative of any of these.
Monoclonal antibodies can be prepared, for example, by the techniques as originally described in Köhler and Milstein, Nature 259 (1975), 495, and Galfré, Meth. Enzymol. 73 (1981), 3, which comprise the fusion of mouse myeloma cells to spleen cells derived from immunized mammals with modifications developed in the art.

As mentioned herein above, the antibodies can be monoclonal antibodies, polyclonal antibodies, chimeric antibodies or synthetic antibodies as well as fragments of antibodies, such as Fab, Fv or scFv fragments etc. Antibodies or fragments thereof can be obtained by using methods which are described, e.g., in Harlow and Lane "Antibodies, A Laboratory Manual", CSH Press, Cold Spring Harbor, 1988. The production of chimeric antibodies is described, for example, in WO 89/09622.

Said antibodies, derivatives and/or fragments thereof may bind to said peptide consisting of a specific tau derived peptide as identified herein above. Therefore, said antibody(ies), said derivative(s) and/or fragment(s) thereof may act as an inhibitor of PHF-formation. In one instance, said antibody(ies) can bind to said peptide and thereby be able to block the interaction of tau protein and/or tau-fragments in vitro and/or in vivo. It is particularly preferred that said antibody, fragment or derivative thereof specifically interacts with the amino acid sequence as depicted in SEQ ID NOs: 7, 8 or 9.

In addition, oligonucleotides and/or aptamers which may specifically bind to the above mentioned peptide(s) and thereby modify and/or inhibit the PHF-formation in vitro and/or in vivo are also envisaged as potential inhibitors to be tested in the method of the present invention. The term "oligonucleotide" as used in accordance with the present invention comprises coding and non-coding sequences, it comprises DNA and RNA and/or comprises also any feasible derivative. The term "oligonucleotide" further comprises peptide nucleic acids (PNAs) containing DNA analogs with amide backbone linkages (Nielson, Science 274 (1991), 1497-1500). Oligonucleotides which may inhibit PHF-formation and which can be identified and/or obtained by the method of the present invention can be, inter alia, easily chemically synthesized using synthesizers which are well known in the art and are commercially available like, e.g., the ABI 394 DNA-RNA Synthesizers.
In accordance with the present invention, the term aptamer means nucleic acid molecules that can bind to target molecules. Aptamers commonly comprise RNA, single stranded DNA, modified RNA or modified DNA molecules. The preparation of aptamers is well known in the art and may involve, inter alia, the use of combinatorial RNA libraries to identify binding sites (Gold, Ann. Rev. Biochem. 64 (1995), 763-797).

Potential other inhibitors to be screened with the method of the present invention include small molecules which bind to, interfere with and/or occupy relevant sites on tau, fragments thereof and/or on peptides which are derived from tau. Furthermore, said inhibitors comprise molecules capable of interfering with amino acid sequences which are derived from the tau protein sequence and consist of the amino acid sequences as depicted in SEQ ID NOs: 6 to 9. In particular said inhibitors interfere with the potential β-sheet formation stretch of peptides as depicted in SEQ ID NOs: 7, 8 or 9. Such inhibitors comprise also molecules which bind to other parts of the full-length tau sequence but inhibit the correct folding of said tau protein (and/or fragments thereof) and thereby inhibit the formation of correct β-structures on and/or within the amino acid sequences as depicted in SEQ ID NOs: 7, 8 or 9. Said molecules might be selected prior to employing the method of the present invention, inter alia, by well known ELISA tests, and/or screening methods like spot membrane binding assays. Further screening methods for "pre-screening" are well known in the art and comprise, inter alia, the screening of phage display libraries, as exemplified, inter alia, in the appended examples. Examples of small molecules include, but are not limited to small peptides (as documented in the appended examples) or peptide-like molecules.

The present invention is particularly useful in assaying for potential PHF-formation inhibitors. It is also envisaged that potential inhibitors, as deduced by other methods known in the art, be further analyzed and/or screened. For example, phage display methods may be employed in order to deduce such potential inhibitors. Said phage display allows the identification of proteins/peptides that interact with a molecule of interest. Such phage display kits are known in the art and commercially available, e.g. Display Systems Biotech # 300-110.

In a preferred embodiment, the invention relates to a method, wherein in step (a) said incubation is carried out in the presence of assembly-competent tau proteins and/or assembly-competent tau protein fragments and wherein in step (b) the presence, decrease and/or absence of PHF or tau filaments is detected. Assembly-competent tau protein(s) and/or fragments thereof may be recombinantly produced or purified. Said proteins or fragments thereof may be derived from different species, e.g., human, mouse, rat, cow, rhesus monkey, or may be derived from human variations of the tau protein, like the FTDP-17 mutations as disclosed in Wilhelmsen (1999), PNAS 96, 7120-7121. Assembly-competent tau proteins are, e.g., full length tau proteins as described in the art, inter alia, in Goedert (1988), Proc. Natl. Acad. Sci. USA 85, 4051-4055; Lee (1988), Science 239, 285-288; Crowther (1994), FEBS letters 337, 135-138; Goedert (1997), Neuroscientist 3, 131 - 141; Goedert, (1989), Neuron 3, 519-526; Himmler, (1989), Mol. Cell. Biol. 9, 1389-1396; Kosik (1989), Neuron 2, 1389-1397; Nelson (1996), J. Neurochem. 67, 1622-1632. Furthermore, assembly-competent tau protein fragments are known in the art and comprise, inter alia, the fragments K11, K12, K18 or K19, as described in Kampers (1996), FEBS letters 399, 344-349. As documented in the appended examples, such a method is particularly useful in screening and/or verifying potential inhibitors of PHF-formation and/or pathological tau-aggregation.

The method(s) of the present invention may be carried out in a physiological buffer system as described, inter alia, in the appended examples. Within the scope of the present invention are furthermore, buffer systems and experimental set-ups, wherein the method(s) of the present invention are carried out in the presence of anionic co-factors for PHF-formation as described herein below. The anionic co-factors comprise, inter alia, heparin, polyglutamic acid, ribonucleic acid. Furthermore, said physiological buffer system may allow oxidation processes, e.g. oxidation of cysteins (see Schweers (1995), P.N.A.S. 92, 8463-8467).

Additionally, the present invention relates to a method for identifying and obtaining an inhibitor of PHF formation comprising the steps of:
(a) contacting a peptide consisting of a specific tau-derived peptide as defined in any one of SEQ ID NOs 6, 7, 8 and 9 with the first molecule known to bind to said peptide to form a first complex of said peptide and said first molecule;
(b) contacting said first complex with a compound to be screened; and
(c) measuring whether said compound displaces said first molecule from said first complex.

Said "first molecule known to bind to said peptide" may comprise, but is not limited to antibodies, antibody fragments, antibody derivatives, aptamers, specific oligonucleotides and/or small molecules which specifically bind to/interact with said peptide as described herein. Said small molecules may comprise, inter alia, synthetically produced peptide-analogs which may comprise, inter alia D-amino acids.
Said "measuring" in step (c) may be carried out by specific immunological and/or biochemical assays which are well known in the art and which comprise, e.g., homogenous and heterogenous assays as described herein below. A further method which may be employed comprises FRET (fluorescence resonance energy transfer; as described, inter alia, in Ng, Science 283 (1999), 2085-2089).

In a preferred embodiment, the present invention relates to the above described method wherein said measuring step (c) comprises the measurement of a formation of a second complex of said tau-derived peptide and said compound.

The measurement of a complex formation is well known in the art and comprises, inter alia, heterogeneous and homogeneous assays. Homogeneous assays comprise assays wherein the binding partners remain in solution and comprise assays, like agglutination assays (see herein below). Heterogeneous assays comprise assays like, inter alia, immuno assays, for example, ELISAs, RIAs, IRMAs, FIAs, CLIAs or ECLs.

Further methods for measurement comprise the monitoring of complex and/or aggregate formation in real time by the fluorescence of dyes such as thioflavine S or T (as described in Friedhoff (1998a, b) loc. cit., or as illustrated in the appended examples), or the measurement of self-fluorescence, like the self-fluorescence of tyrosine (as illustrated in the appended examples).

In a more preferred embodiment, the method of the invention is a method wherein said measuring step (c) comprises the measurement of the amount of said first molecule that is not bound to said tau-derived peptide.

Again said measurement may comprise a plurality of measuring and/or detection methods which are well known in the art. For example, said first molecule and therefore said amount may be measured by immuno detection methods or by the detection of a label which was added to said first molecule. Said label may be, inter alia, a tag, a fluorescent marker, an enzyme, a peptide or a radioactive marker.

Such labels are well known to the person skilled in the art and comprise, inter alia, horse radish peroxidase, alkaline phosphatase, β-galactosidase, fluorochromes (like fluorescein, rhodamine, Texas Red, etc.), colloidal metals (like gold particles), biotin, digoxygenin and chemi-or bioluminescent compounds.

Any measuring or detection step of the method(s) of the present invention may be assisted by computer technology. For example, in accordance with the present invention, said detection and/or measuring step can be automated by various means, including image analysis, spectroscopy or flow cytometry.
In a particularly preferred embodiment, said measurement/detection is carried out in a fluorescence assay employing, inter alia, thioflavin S or T (see herein above) or tyrosine self-fluorescence. Such assays are well known in the art, as illustrated in the appended examples and/or as described in Friedhoff (1998a, loc. cit.). Said tyrosine self-fluorescence is partially useful for measurements and/or detections of assembly, aggregation, dimerization processes, or the like, where small peptides/proteins are involved which comprise an intrinsic tyrosine. For example, the presence and/or formation of aggregation products of peptides consisting of a specific tau-derived peptide as defined, inter alia, in any one of SEQ ID NOS: 6, 7, 8, or 9 can be readily measured, as demonstrated in the appended examples.

Therefore, the detection/measuring step(s) of the method(s) of the invention can be easily performed according to methods known in the art such as described herein or as demonstrated in the appended examples. In particular, the detection/measuring step(s) of the method(s) of the invention can be carried out by employing antibodies directed against said tau derived peptides. Said antibodies may comprise conformation-dependent antibodies. The use of antibodies is particularly preferred in detection methods like ELISA.

The compounds/inhibitors identified and/or obtained according to the method of the invention, in particular inhibitors interacting with said peptides comprising a specific tau derived peptide as defined herein or (a) fragment(s) thereof, are expected to be very beneficial as neuroprotective agents. The inhibitors identified are thus expected to be very useful in diagnostic and in particular for therapeutic applications.

Thus, in addition, the present invention relates to a method for the preparation of a pharmaceutical composition comprising the steps of a method described herein above and synthesizing and/or formulating the compounds identified, obtained and/or screened in step (b) or derivative thereof in a pharmaceutically acceptable form. The therapeutically useful inhibitor/compound identified according to the method of the invention may be formulated and administered to a patient as discussed herein below. For uses and therapeutic doses determined to be appropriate by one skilled in the art, see infra.

In a more preferred embodiment, the method of the invention relates to a method wherein said peptide comprising a tau-derived peptide is bound to a solid phase.

The method of the invention is preferably carried out in the liquid phase, however, said peptide can be bound to a solid phase/a solid carrier. Additionally, as pointed out herein above, said peptide can be additionally labeled in various ways, inter alia, by fluorescence or radio labels. Said solid phase comprises, but is not limited to, a plastic, a glass, a silicon, a colloidal metal, a cellulose and/or a polymeric support. Said solid support is selected from the group consisting of solid organic polymers, cellulose/cellulose-based membranes, colloidal metal particles, plastic surfaces, or any combination thereof. A number of supports known in the art are suitable for serving the purposes of the present invention. Such supports may comprise, inter alia, membranes, plates, stripes, wells, beads, microchips or containers. Suitable materials for such supports or materials for further coating of said supports include, but are not limited to, glass, polystyrene, polyvinyl chloride, polypropylene, polyethylene, polycarbonate, latex, dextran, nylon, amyloses, natural and modified celluloses, like nitrocellulose, polyacrylamide, agaroses, magnetide and metals. The above mentioned colloidal metal particle may be, inter alia, a gold particle, and said above mentioned plastic surface comprises the well of a microtiter plate. Additionally, said above mentioned solid organic polymer may be or comprise a latex bead. Combinations of several supports, such as, e.g. latex beads and colloid metal particles, are also within the scope of the present invention.
Said particles bound to solid supports/phases are useful in the method of the present invention when, inter alia, said detection and/or measuring step is carried out by homogeneous assays, for example by an agglutination assay which measures the formation of a complex of said peptides. An example of such a homogeneous assay would be a latex enhanced turbimetric assay. In accordance with the present invention it might be envisaged that either the direct interaction of a peptide consisting of a specific tau derived peptide as defined in SEQ ID NOs: 6, 7, 8, or 9 with another of said peptides is measured or that at least one of the binding/complex partners is/are bound to the above described solid phases/supports, such as latex beads, and that the interaction/agglutination of said solid support is measured. Such a measurement is known to the person skilled in the art and comprises, inter alia, electric field variation, magnetic field variation, turbidimetric variation and/or light scattering. Said measurement, however, may also involve adapted methods as described herein and in the appended examples, like fluorescence assays employing thioflavin S or T or self-fluorescence of tyrosine.

In a particular preferred embodiment, the method of the present invention comprises a method, wherein the incubation step involving a peptide comprising a specific tau derived peptide as defined herein is carried out in the presence of (poly)anions. Said (poly)anions may comprise, but are not limited to, heparin, nucleic acid molecules, or acidic peptides (like poly-Glu).

In accordance with the present invention, the method of the present invention also comprises the use of in vivo-test systems. It is, inter alia, envisaged that transgenic animals comprise peptides as defined herein, i.e. peptides consisting of specific tau derived peptides as defined in any one of SEQ ID NOS: 6. 7, 8, and 9. Said transgenic animal preferably expresses the "minimal tau peptide" as defined herein in the nervous system, however, expression in other organs of said transgenic animal are within the scope of the present invention.

Furthermore, it is envisaged that the minimal tau peptide as defined in the present invention (SEQ ID NOS. 6 to 9) is expressed in tissue and/or cell cuture systems. Said tissue and/or cell culture systems may comprise primary as well as secondary tissue culture systems. For example, cultured hippocampal neurons, cultured neuronal stem cells, cultured (embryonic) dorsal root ganglia or cultured spinal cord, may be employed in the method of the present invention. Cell systems comprise, but are not limited to, neuronal cells like PC12-cells, NT2, N-A or non-neuronal N2A cells, like HEK293, CHO, Hela, NIH3T3.

The above-mentioned tissue and/or cell cultures may for example be transfected with nucleic acid molecules encoding the minimal peptide as defined herein. Transfection systems are well known in the art and comprise but are not limited to CaCl₂-transfections, transfections employing lipofectamine, viral transfection systems, like adenoviral or lentiviral systems, and the like.

In full length tau isoforms and tau-derived peptides, site-directed mutagenesis of the hexapeptide as shown in SEQ ID NOS 7 or 8 that inhibits its beta-structure forming capacity also inhibits aggregation (for example, replacement of V, Q, I by N, A, E , deletion of VQI, or exchange of single residues by a proline). In cell models and transgenic animal models the use of such mutations in the hexapeptide PHF6 is therefore particularly useful to determine whether tau aggregation per se is toxic (gain of toxic function), or whether other properties of tau play a role in degenerating neurons of Alzheimer's disease (e.g. destabilization of microtubules after loss of tau-microtubule binding, or inhibition of intracellular transport, i.e. loss of function). Furthermore, proteins/peptides comprising (a) mutated and/or modified version(s) of the hexapeptide as defined in SEQ ID NOS 7 or 8 may be particularly useful in drug screening assays and/or may be employed as inhibitors of β-sheet formations.

Furthermore, the present invention relates to an inhibitor identified or obtained by the method described herein. In a preferred embodiment, said inhibitor specifically binds to a peptide comprising a tau-derived peptides as defined in any one of SEQ ID NOS: 6, 7, 8 or 9.
The term "specifically binding" as used in accordance with the present invention means "specifically interacting" with. Said interaction may be, inter alia, covalently, non-covalently and/or by hydrophobic interaction. As inhibitors, as identified or obtained by the method to the present invention are envisaged small molecule peptides, antibodies, aptamers and the like. It is particularly preferred that said inhibitor is capable fo breaking and/or interfering with the β-structure of the minimal tau-peptide as defined herein or which are capable fo breaking and/or interfering with the β-sheet like interaction of tau.

The invention relates in addition to a composition comprising the inhibitor as identified or obtained by the method as described herein above or the inhibitor which specifically binds to a peptide comprising a tau derived peptide as defined herein above or (a) fragment(s) thereof. In a preferred embodiment, said inhibitor is an antibody, as described herein above. In a particularly preferred embodiment, said antibody is a monoclonal antibody. As mentioned herein above, said inhibitor also comprises small molecule peptides, aptamers and/or compounds capable of interfering with a β-sheet formation.

The term "composition" as used in accordance with the present invention comprises at least one inhibitor as identified or obtained by the method of this invention or at least one inhibitor which specifically binds to a peptide consisting of a tau derived peptide as defined herein above and, optionally, further molecules, either alone or in combination, like e.g. molecules which are capable of interfering with the formation of neurofibrillary tangles and/or the formation of amyloid plaques. The composition may be in solid, liquid or gaseous form and may be, inter alia, in a form of (a) powder(s), (a) tablet(s), (a) solution(s) or (an) aerosol(s). In a preferred embodiment, said composition comprises at least two, preferably three, more preferably four, most preferably five inhibitors as defined herein above.

In a preferred embodiment, said composition is a diagnostic or a pharmaceutical composition.

The pharmaceutical composition may further comprise a pharmaceutically acceptable carrier, excipient and/or diluent.
Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g. by intravenous, intraperitoneal, subcutaneous, intramuscular, topical, intradermal, intranasal or intrabronchial administration. Most preferably, said administration is intra-cerebral and/or by an administrative route which by-passes the blood/brain barrier. The dosage regimen will be determined by the attending physician and clinical factors. As is well known in the medical arts, dosages for any one patient depends upon many factors, including the patient's size, body surface area, general health, age, sex, the particular compound to be administered, time and route of administration, and other drugs being administered concurrently. Pharmaceutically active matter may be present, inter alia, in amounts between 1 ng and 100 mg per dose; however, doses below or above this exemplary range are envisioned, especially considering the aforementioned factors.
The compositions of the invention may be administered locally or systemically. Administration will generally be parenterally, e.g., intravenously. The compositions of the invention may also be administered directly to the target site, e.g., by biolistic delivery to an internal or external target site (like the brain) or by catheter to a site in a (brain) artery. Furthermore, the pharmaceutical composition of the invention may comprise further agents, depending on the intended use of the pharmaceutical composition. Such agents may be drugs acting on the central nervous system, like neuroprotective factors, cholinesterase inhibitors, agonists of M1 muscarinic acetylcholine receptor, drugs leading to increased synthesis of acetylcholine, as well as non-steroid antiinflammatory drugs (NSAIDs), hormones, antioxidants, inhibitors of the production and/or aggregation of Aβ, or inhibitors of the production, aggregation and/or phosphorylation of tau. Further drugs/agents which may be compised in the pharmaceutical composition of this invention comprise any Alzheimer-relevant medication as, e.g., described under www.alzforum.org/members/research.

In a preferred embodiment of the invention, the composition of the present invention is a diagnostic composition further comprising, optionally, suitable means for detection. The diagnostic composition comprises at least one of the aforementioned inhibitors of the invention.

The pharmaceutical compositions, as well as the methods of the invention described supra or the uses of the invention described infra can be used for the treatment of all kinds of diseases hitherto unknown or being related to or dependent on pathological tau aggregation. They may be particularly useful for the treatment of Alzheimer's disease and other diseases where intracellular deposits of tau, like frontotemporal dementia, FTDP-17-mutations (Wilhelmsen (1999), loc. cit) and other tauopathies, appear to play a role. They may be desirably employed in humans, although animal treatment is also encompassed by the methods, uses and compositions described herein.

In yet another embodiment, the present invention relates to a method for detecting and/or measuring PHF formation comprising the steps of:
(a) incubating a peptide consisting of a specific tau derived peptide as defined in any one of SEQ ID NOs 6, 7, 8 or 9, with tau-proteins and/or fragments thereof under conditions which permit assembly of tau-proteins and/or fragments thereof into PHFs; and
(b) detecting the presence, absence, decrease or increase of PHFs and/or nucleation sites of PHF assembly.

The incubation(s) of step (a) in the method(s) of the present invention may be carried out in vitro solely in the presence of tau-proteins and/or fragments thereof. However, further co-factors which might promote PHF assembly/formation and/or formation of nucleation sites for PHF assembly may be added. Such co-factors are well known in the art and comprise, e.g., anionic co-factors, like polyglutamate, RNA or heparin (see, inter alia, Friedhoff (1998a), loc. cit.). The detection/measuring step (b) may be carried out as described herein and in the appended examples and may comprise microscopic and/or spectroscopic methods.

In a more preferred embodiment, the detection and/or measuring step in the method of the present invention is carried out by microscopic or spectroscopic means. In a yet more preferred embodiment said microscopic means comprises electron microscopy and said spectroscopic means comprises circular dichroism spectroscopy, fluorescence spectroscopy or infrared spectroscopy (FTIR). In a particular preferred embodiment said fluorescence spectroscopy comprises a thioflavine S or T assay or a tyrosine self-fluorescence-assay, as described herein. For example, in said tyrosine self-fluorescence assy, the change in fluorescence of Tyr 310 (within the β-structure forming hexapeptide of the present invention) of tau changes (see, inter alia, appended examples VI). Said microscopic and/or spectroscopic means are well known in the art as illustrated, inter alia, in the appended examples. Further detection methods are described in the art and comprise methods as described in the appended examples. As mentioned herein above, suitable methods comprise not only microscopic and/or spectroscopic means but comprise also heterogeneous or homogeneous assays, like ELISAs or agglutination assays.

In yet another embodiment, the present invention relates to a kit comprising:
(a) a peptide consisting of a specific tau derived peptide as defined in any one of SEQ ID NOs 6, 7, 8 and 9 and/or a nucleic acid molecule encoding said peptide or a solid support as defined herein above; and
(b) tau-protein(s) and/or fragments thereof as defined herein above;
adopted for carrying out the method of the invention, optionally further comprising a standard and/or suitable means for detection.

Furthermore, the present invention relates, in another embodiment, to a kit comprising an inhibitor or a composition as defined herein above. Such (a) kit(s) of the present invention may comprise a carrier means being compartmentalized to receive in close confinement one or more container means such as vials, tubes and the like, each of the container means comprising one of the separate elements to be used in the method of the invention or for the composition of the invention. For example, one of the container means may comprise the peptide comprising a tau-derived peptide as defined herein, soluble or bound to a carrier. A second container may comprise soluble tau protein(s) or (a) fragment(s) thereof, in lyophilized form or in solution. In addition, the carrier means may also contain a plurality of containers each of which comprises different, predetermined amounts of peptides as defined herein above. These latter containers can then be used to prepare a standard curve into which can be interpolated the results obtained from the methods of the invention identifying and obtaining an inhibitor capable of modifying PHF formation in vitro and/or in vivo.

The kit(s) of the invention is (are) particularly useful in carrying out the method of the invention that has been described in detail herein above. In a further embodiment, the present invention relates to the use of a peptide comprising a specific tau derived peptide as defined herein above for carrying out the method(s) of the invention for identifying and obtaining an inhibitor, capable of modifying the PHF formation in vitro and/or in vivo, for the preparation of a pharmaceutical composition and/or for detecting and/or measuring PHF formation in vitro.

A particularly preferred embodiment of the invention relates to the use of the kit(s) of the invention for carrying out the methods of the invention.

Furthermore, the present invention relates to the use of a peptide consisting of a specific tau derived peptide as defined in SEQ ID Nos: 6 to 9 and/or a nucleic acid molecule encoding said peptide for carrying out the method of the present invention and/or for screening and/or for the identification of an inhibitor, capable of modifying PHF formation. These screening and/or identification methods comprise not only methods wherein amino acid sequences, as depicted, inter alia, in SEQ ID NOs: 6 to 9, are used but also relate to methods wherein nucleic acid molecules encoding these peptides are employed. Proteins and other compounds which bind to the peptides comprising tau derived peptides as described in the present invention are candidates or inhibitors of PHF formation according to the present invention. Such binding compounds can be "captured" using the yeast two-hybrid system (Fields and Song (1989), Nature 340, 245-246).
Therefore, and in context of the method and use as described herein, it is also envisaged that nucleic acid molecules encoding the minimal tau peptide as defined herein, may be employed in order to carry out the methods of the present invention or may be employed in the use of the present invention.
A modified version of the yeast two-hybrid system has been described by Roger Brent and his colleagues (Gyuris, J. et al. (1993), Cell 75, 791-803; Zervos, A.S. (1993), Cell 72, 223-232). Preferably, the yeast two-hybrid system is used according to the present invention to capture compounds which bind to the peptides as identified in SEQ ID Nos: 6 to 9. Such compounds are good candidate inhibitors of PHF formation of the present invention. Other screening and/or identification methods are well known in the art and comprise, inter alia, phage display (as described, inter alia in US 5,541,109) or affinity-labelings via cross-linkers. Furthermore, the method of the present invention is therefore also useful in and can easily be adapted to high-throughput screenings and/or computerized screening assays.

The present invention also describes a non-human transgenic animal expressing the minimal tau peptide as defined hereinabove. Most preferably, said transgenic animal comprises and/or expresses the minimal tau peptide as defined in. SEQ ID NOS. 6 to 9 or a functional homologue thereof. Said transgenic animal may, inter alia, be selected from the group consisting of mouse, rat and sheep.
Yet, it is also envisaged that said transgenic animal comrpises and/or expresses a minimal tau peptide and/or tau peptide and/or (a) fragment(s) of tau which comprise a hexapeptide as defined in SEQ ID NOS 7 or 8, wherein at least one amino acid with intrinsic β-sheet propensities(like, inter alia, V, Q, I) has been replaced with (an) amino acid(s) which do not comprise such intrinsic β-sheet propensities or comprise limited intrinsic β-sheet propensities (like, inter alia, N, E, A, P).
Accordingly, the present invention also relates to the uses and methods as disclosed herein, wherein the peptide comprising a specific tau-derived peptide as defined in any one of SEQ ID NOS 6 to 9 further comprises in the hexapeptide as defined in SEQ ID NOS 7 or 8 (and which are comprised in SEQ ID NOS 6 or 9) at least one, at least two, at least three or at least four mutations and/or modifications. Said mutation and/or modification may comprise exchanges, substitutions, additions and/or deletions. It is particularly preferred that, depending on the described uses and methods, said mutations and/or modifications comprise the replacement of amino acids with high intrinsic (β-sheet propensity(ies) with amino acids with similarly high intrinsic β-sheet propensity(ies) (e.g., replacement of V, Q and/or I, with, e.g. T, F and/or Y) or the replacement with amino acids with lower or no intrinsic β-sheet propensity(ies) (e.g., replacement of V, Q and/or I with N, A and/or E or the exchange of at least one residue by proline). Therefore, the present invention also relates to uses and methods as disclosed herein wherein the above mentioned mutation and/or modification comprises the replacement of at least one amino acid in the hexapeptide as depicted in SEQ ID NOS. 7 or 8 (or in proteins/peptides comprising said hexapeptide).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

The figures show:

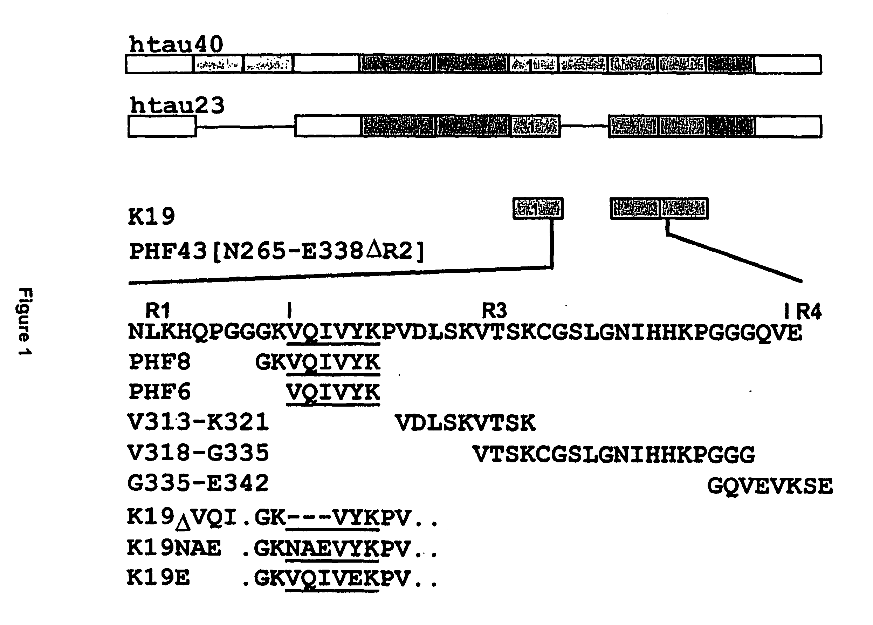

**Fig. 1 Bar diagram of tau constructs and peptides.** (a) htau40, the longest isoform in the human CNS, containing four repeats of -31 residues (labeled 1-4) and two inserts near the N-terminus. Residue numbering follows that of htau40 throughout. (b) htau23, the smallest and fetal isoform, lacking the N-terminal inserts and repeat R2. (c) K19 represents only the repeat domain of htau23, i.e. R1, R3, R4 (i.e. 94 residues, Q244-N368, but without V275-S305). (d) PHF43, a subfragment of K19, containing the end of R1, all of R3, and the beginning of R4, but without R2 (43 residues). (e) PHF8, 8 residues spanning the junction between R1 and R3. (f) PHF6, 6 residues from the beginning of R3. The other peptides (V318-K321, V318-G335 and G335-E342) span the PHF43 sequence and were used for analysis by CD.

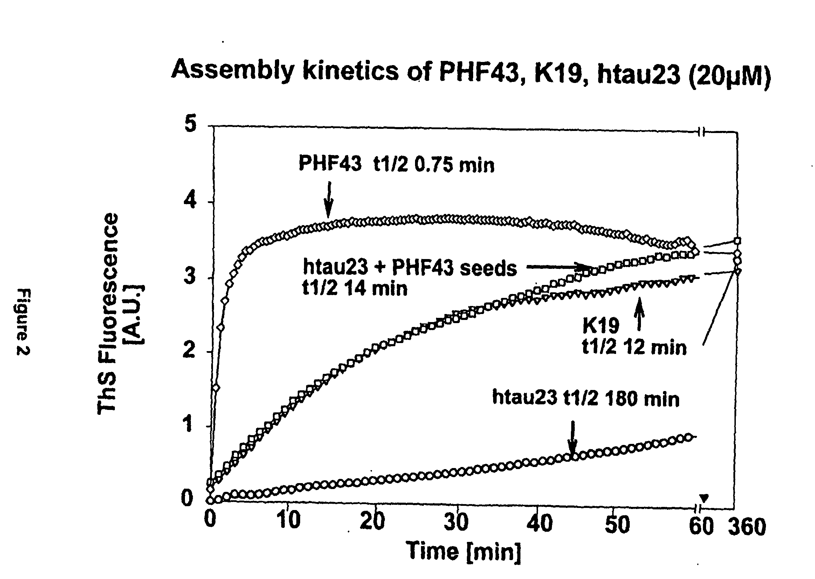

**Fig. 2: Kinetics of PHF assembly.** The assembly of tau or tau fragments was measured fluorimetrically, using the fluorescence of ThS (Friedhoff et al., 1998a). Protein concentrations were 20µM, in the presence of 5µM heparin and 20 mM NH₄Ac, at room temperature. PHF43 assembles rapidly and spontaneously (diamonds, top curve, t_{1/2} = 0.75 min), but htau23 is very slow by comparison (circles, bottom curve, t_{1/2} = 180 min). K19 shows intermediate kinetics (triangles, t_{1/2} = 12 min). Htau23 can be speeded up to a similar rate as K19 in the presence of seeds from sonicated fibers obtained after PHF43 assembly (squares, t_{1/2} = 14 min). The K19 mutants (K19^{ΔVQI}, K19^{NAE} and K19^{Y310E}) showed no aggregation even after several hours (filled triangle at bottom right).
**Fig. 3: Peptides derived from tau and their ability for PHF assembly.**
   The peptide K19 contains the repeats 1, 3, and 4 of tau, but repeat 2 (exon 10) is absent, as in the fetal human isoform tau23 (see Fig. 1). In the standard numbering according to htau40, the longest isoform in the human CNS, K19 extends from residue 244 to 368, but lacking the 31 residues 275-305 (which in 4-repeat isoforms would be inserted after K275, labeled with an asterisk). Each repeat terminates with a characteristic PGGGX motif. Mutations of K19 (K19^{ΔVQI}, K19^{NAE} and K19^{Y310E}) are indicated below the K19 sequence. Chymotrypsin cleaves K19 after Y310 and F345 and thus cuts the minimal interaction motif in two parts. GluC at pH 4.0 cleaves K19 after E264, E337 and E342, leading to four fragments as defined in SEQ ID NOS: 52, 6, 53 and 54. This generates the 43 residue peptide PHF43 (underlined, N265-E337 without the second repeat; see also SEQ ID NO:6). GluC at pH 7.8 cleaves K19 after D252, E264, D314, and E337, leading to five fragments as depicted also in SEQ ID NOS:55 to 59. It cleaves PHF43 into two halves but leaves the minimal interaction motif intact (see also SEQ ID NO:57). Other synthetic peptides are listed below: PHF6 (SEQ ID NO: 8), PHF8 (SEQ ID NO: 9), V313-K321 (SEQ ID NO: 17), V318-G335 (SEQ ID NO: 18), G335-E342 (SEQ ID NO: 19). As demonstrated in the appended examples, fragments comprising the minimal VQIVYK motif (SEQ ID NO:8) are capable of specific aggregation (asterisk) and/or the formation of nucleation sides for PHF formation (+).

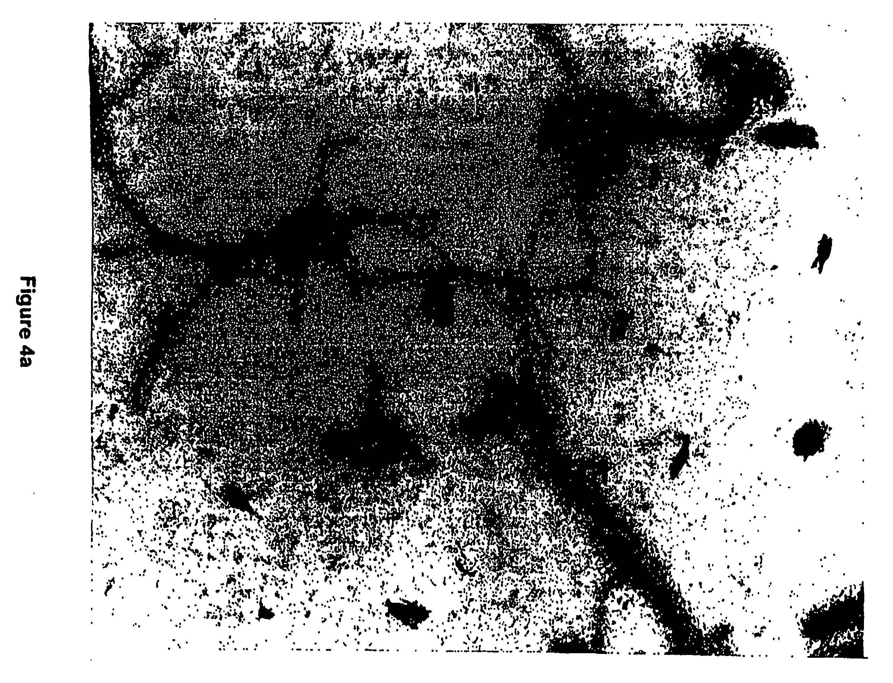

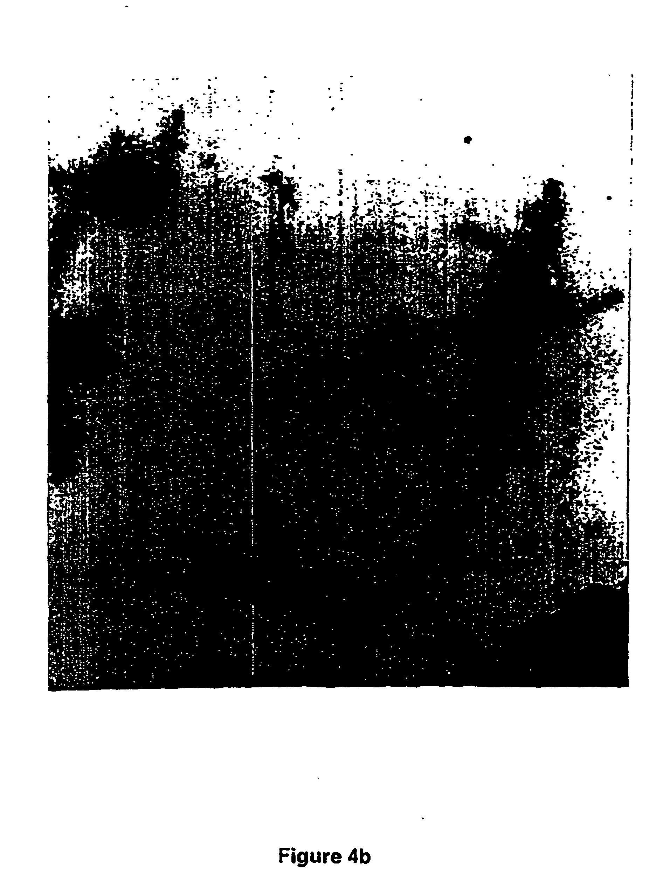

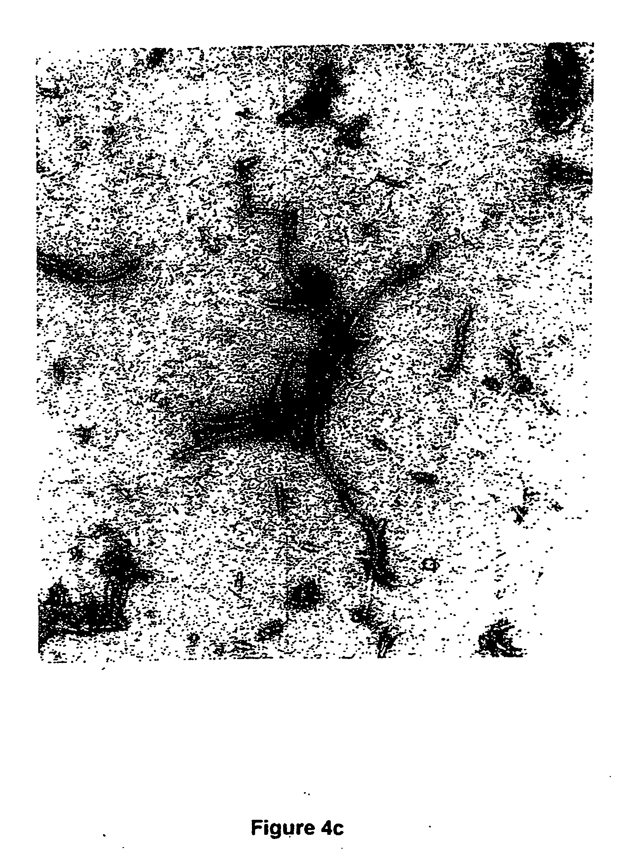

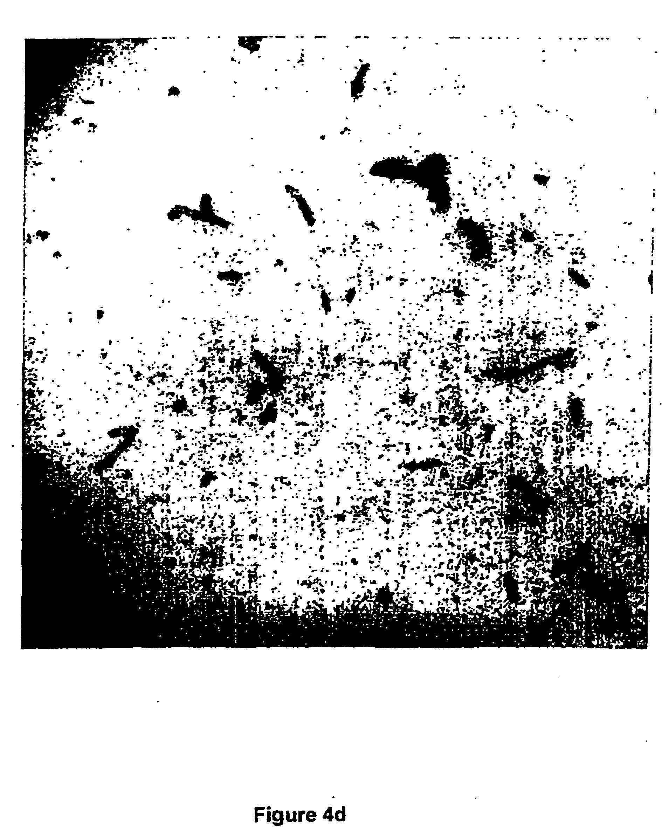

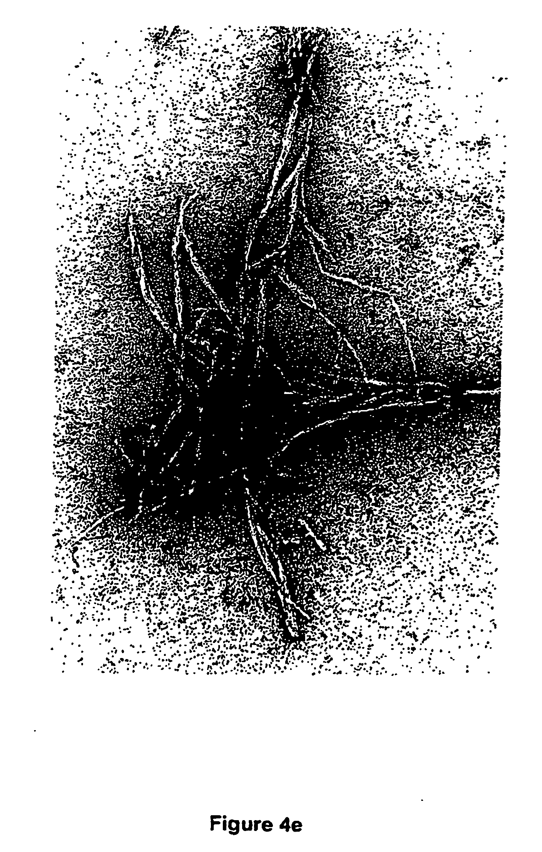

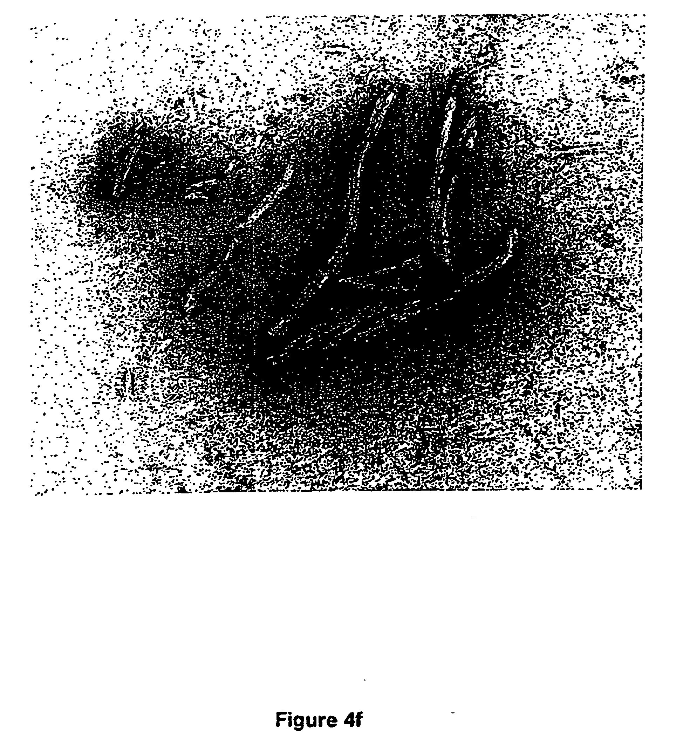

**Fig. 4: Electron micrographs of fibers obtained from the self-assembly of tau or tau peptides**. Assembly conditions were the same as in Fig. 2 (20 µM protein, 5 µM heparin), except E and F (660 µM peptide, 660 µM heparin). A-C show mostly twisted filaments (width ~10-20 nm) resembling Alzheimer PHFs polymerized from (A) htau23, (B) htau23 plus seeds made from PHF43, (C) PHF43. (D) shows the seeds obtained by sonication of PHF43 fibers. (E) and (F) show filamentous aggregates with variable diameters obtained from the short peptides PHF8 and PHF6.

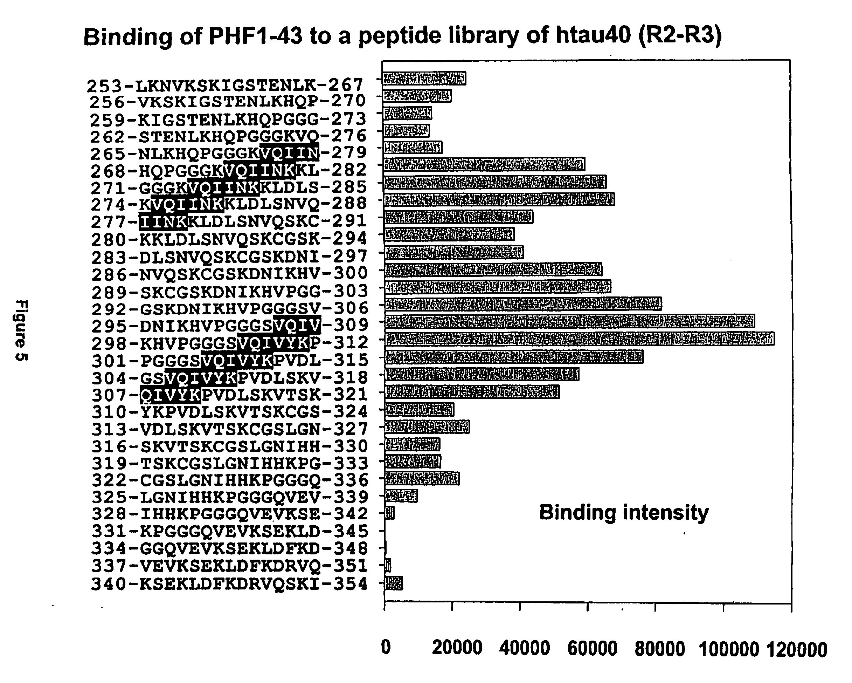

**Fig. 5: Peptide spot membrane interactions between PHF43 and peptides derived from the repeat region of tau.**
   The region of tau from L253 in repeat 1 to D348 in repeat 4 (encompassing the sequence of PHF43) was subdivided into consecutive 15-mer peptides staggered by 3 residues, synthesized and covalently attached to a cellulose membrane (Frank, J. Biotechnol. 41 (1995), 259-272), and incubated with iodinated PHF43. The bars represent the bound radioactivity determined by autoradiography. The strongest interaction occurs around spot 4-25 which contains the sequence of PHF6 (VQIVYK, bold) at the beginning of R3. Another cluster of strong interaction occurs around spot 4-16 which harbors the analogous sequence in R2 (VQIINK, bold).

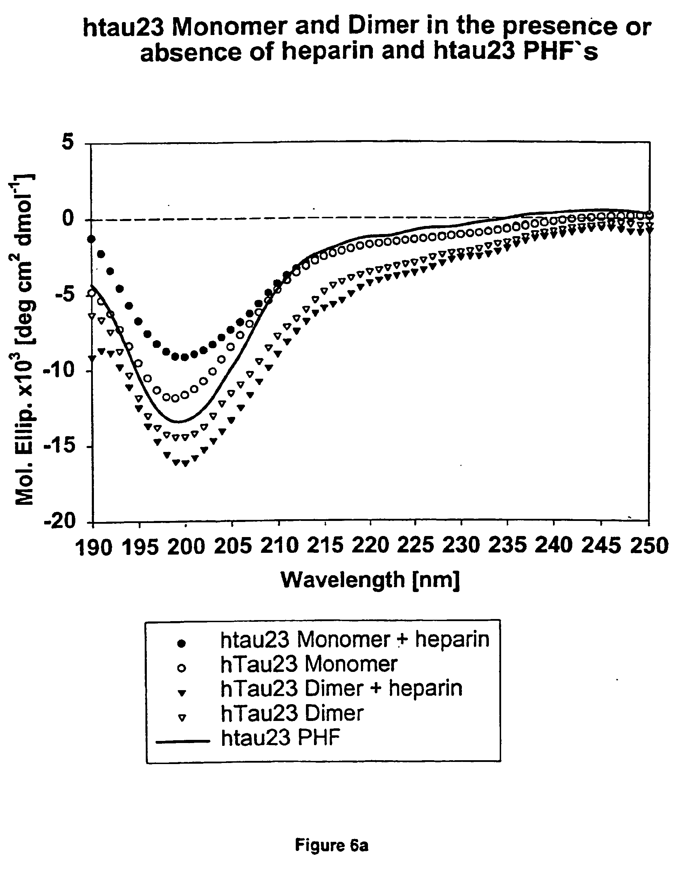

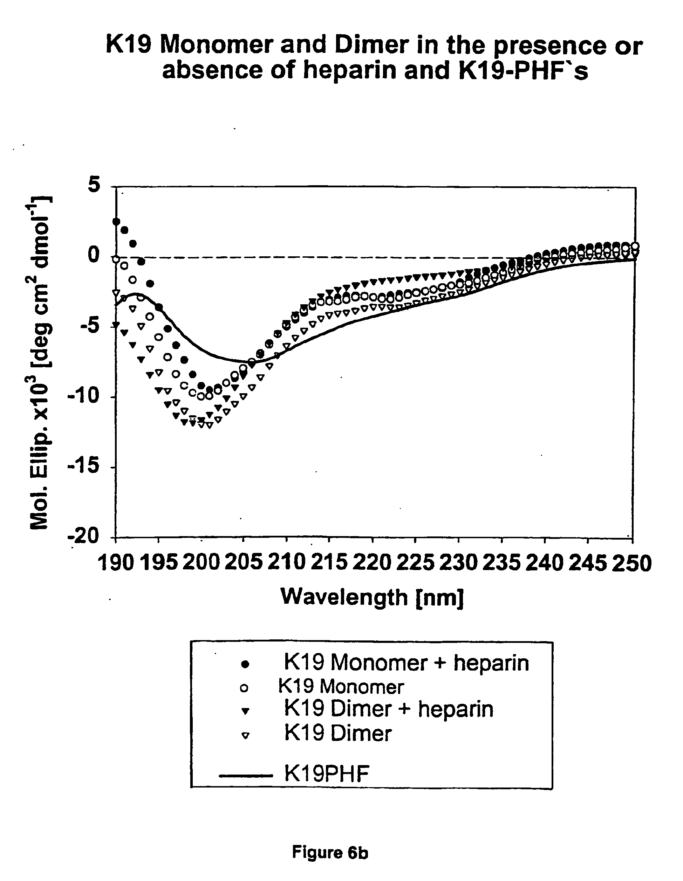

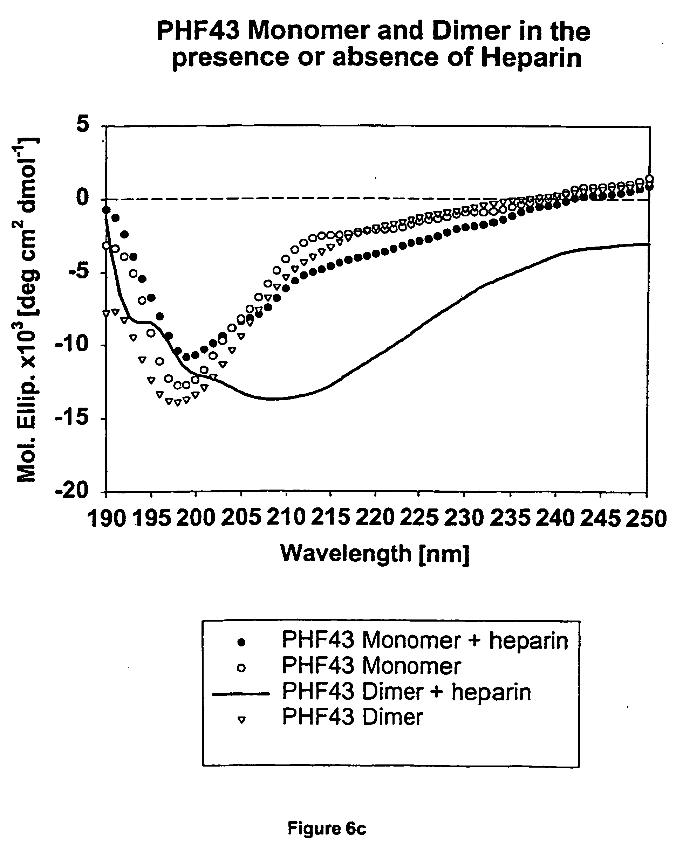

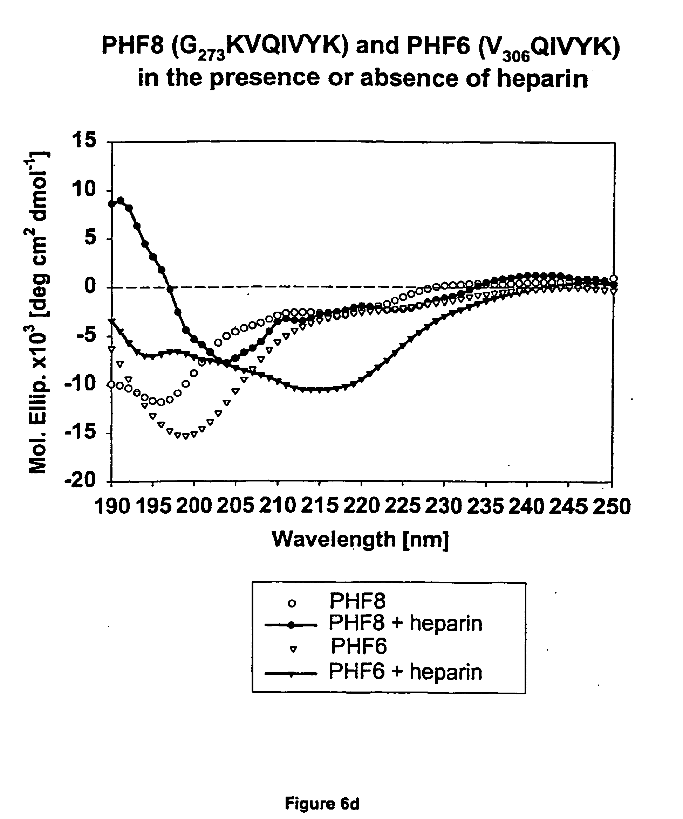

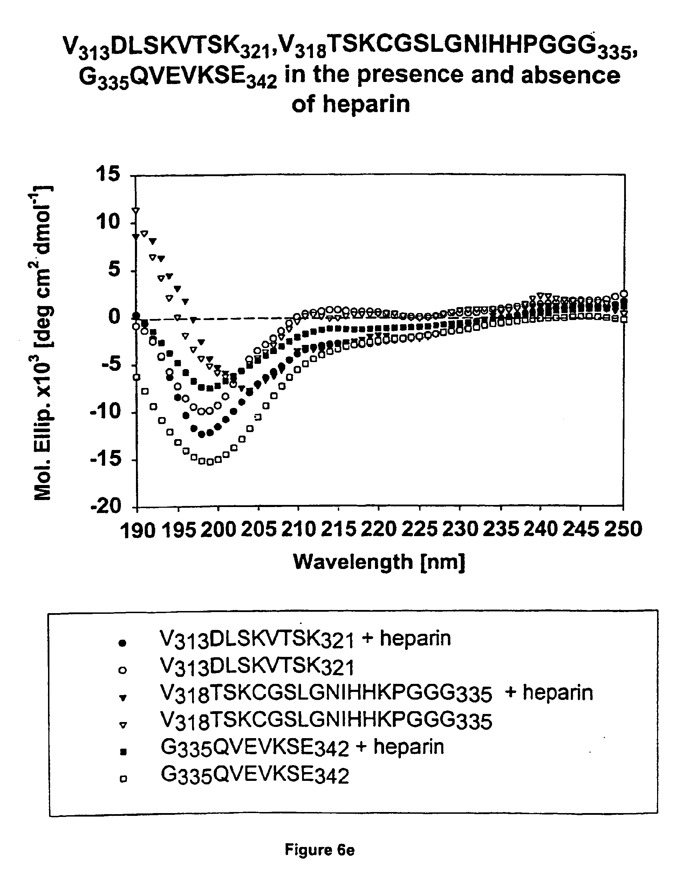

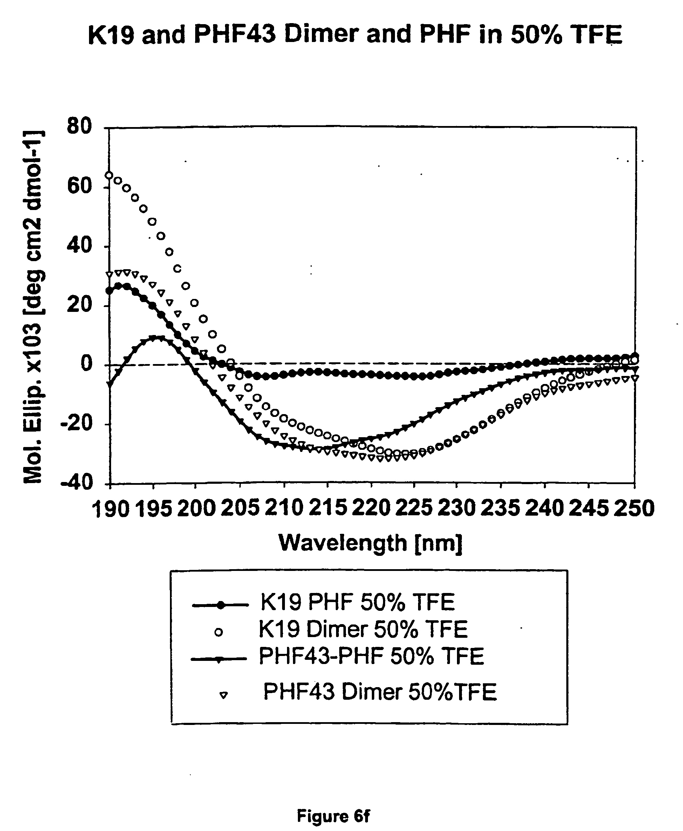

**Fig. 6: CD spectroscopy of tau constructs and tau polymers**. CD spectra were obtained at 50 µM protein concentrations in 10 mM NH₄-acetate at room temperature, in the absence or presence of 50 µM heparin and/or 50% TFE.
   (a) htau23 monomers (circles), dimers (triangles), and assembled PHFs (solid line) show similar CD curves with or without heparin (filled or open symbols). The minimum is around 200 nm, indicating that a random coil structure predominates independently of dimerization or polymerization.
   (b) K19 monomers (SEQ ID NO: 10; without heparin open circles, with heparin filled circles) and dimers (without heparin open triangles, with heparin filled triangles) show similar CD curves, with a mostly random coil structure (minimum around 200 nm), except after polymerization with heparin (solid line) which indicates an increase in beta sheet structure.
   (c) PHF43 monomers (SEQ ID NO: 6; without heparin open circles, with heparin filled circles) and dimers without heparin (open triangles) show mostly random coil structure, but a noticeable shift to beta sheet structure when dimers are assembled into PHFs in the presence of heparin (solid line).
   (d) PHF8 (SEQ ID NO: 9; without heparin open circles, with heparin filled circles) and PHF6 (SEQ ID NO: 8; without heparin open triangles, with heparin filled triangles) show mostly random coil structure, but shift to more beta sheet structure (especially PHF6) upon polymerization in the presence of heparin (compare Fig. 2).
   (e) Three peptides (V₃₁₃DLSKVTSK₃₂₁, SEQ ID NO: 17; without heparin open circles, with heparin filled circles, V₃₁₈TSKCGSLGNIHHKPGGG₃₃₅ SEQ ID NO: 18 without heparin open triangles, with heparin filled triangles, G₃₃₅QVEVKSE₃₄₂, SEQ ID NO: 19; without heparin open squares, with heparin filled squares) derived from PHF43 (but not including the sequence of PHF8) show mostly random coil structure, little dependence on heparin, and no polymerization.
   (f) K19 (open circles) and PHF43 dimers (open triangles) and PHFs (K19, filled circles, PHF43, filled triangles) in the presence of 50% TFE. This tends to induce alpha-helical conformation, most visible in K19 dimer but less in K19-PHFs which are restricted in their conformation. A similar change is observed with PHF43.

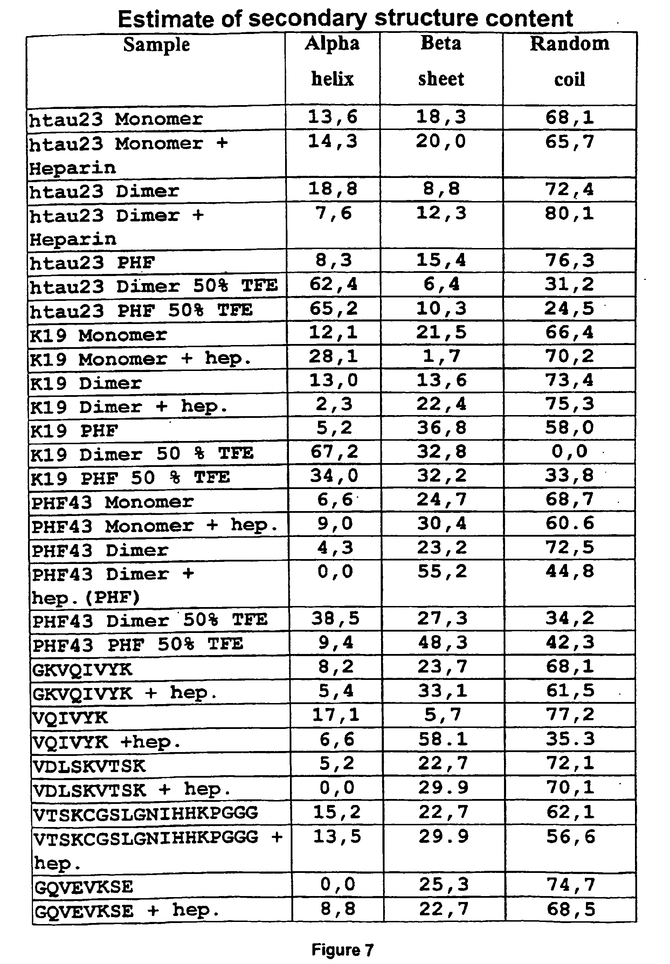

**Fig. 7: Secondary structure content of tau derived peptides.** Secondary structure components of tau constructs and peptides in monomeric, dimeric, and polymeric states, without or with 50% TFE. The secondary structure assignment was calculated from the CD spectra according to Greenfield and Fasman (Greenfield and Fasman, Biochemistry 8 (1969), 4108-4116).

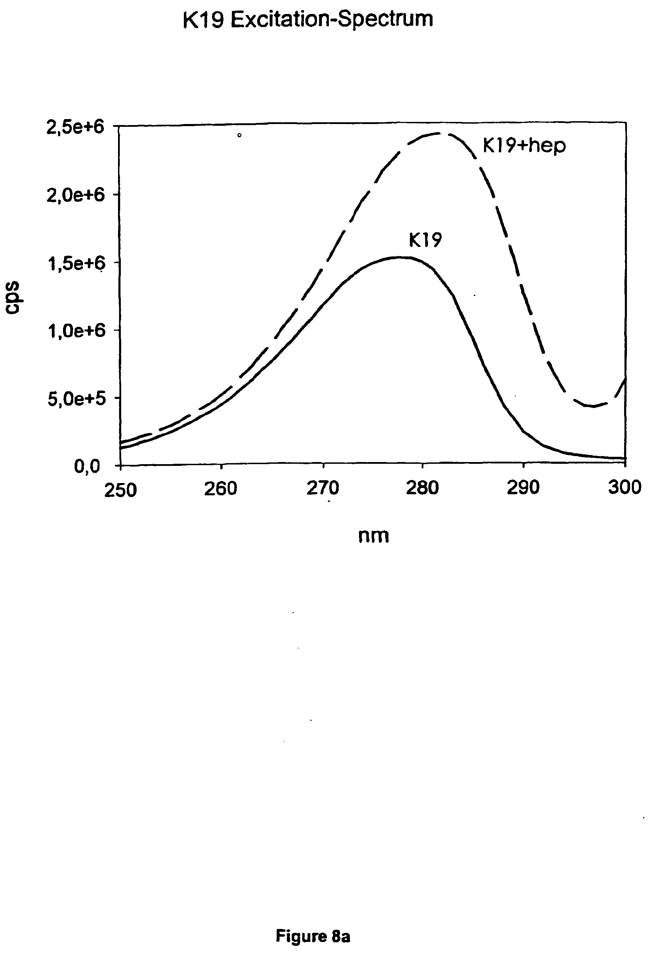

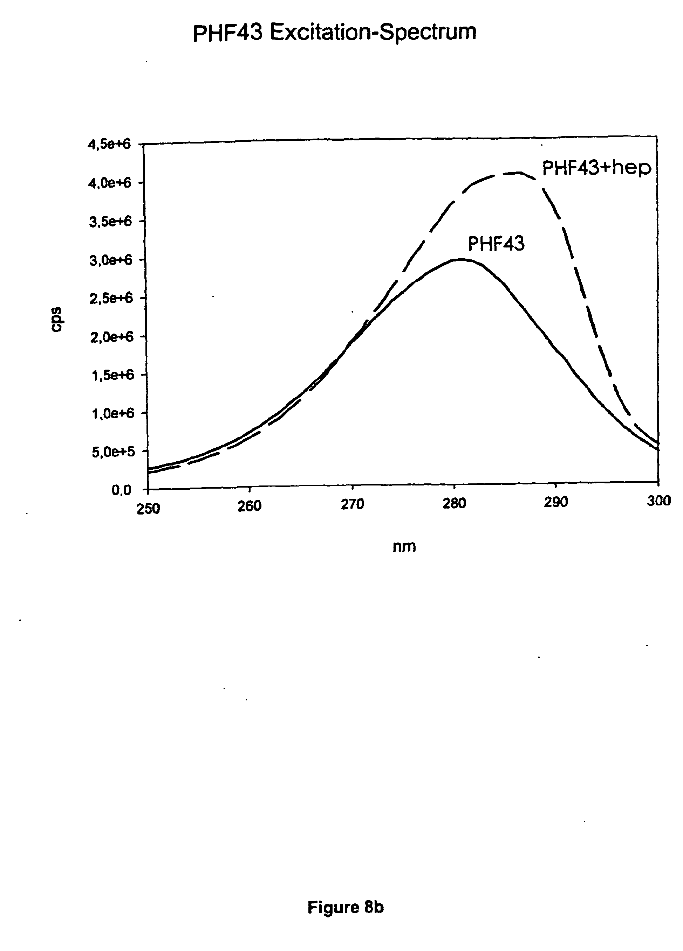

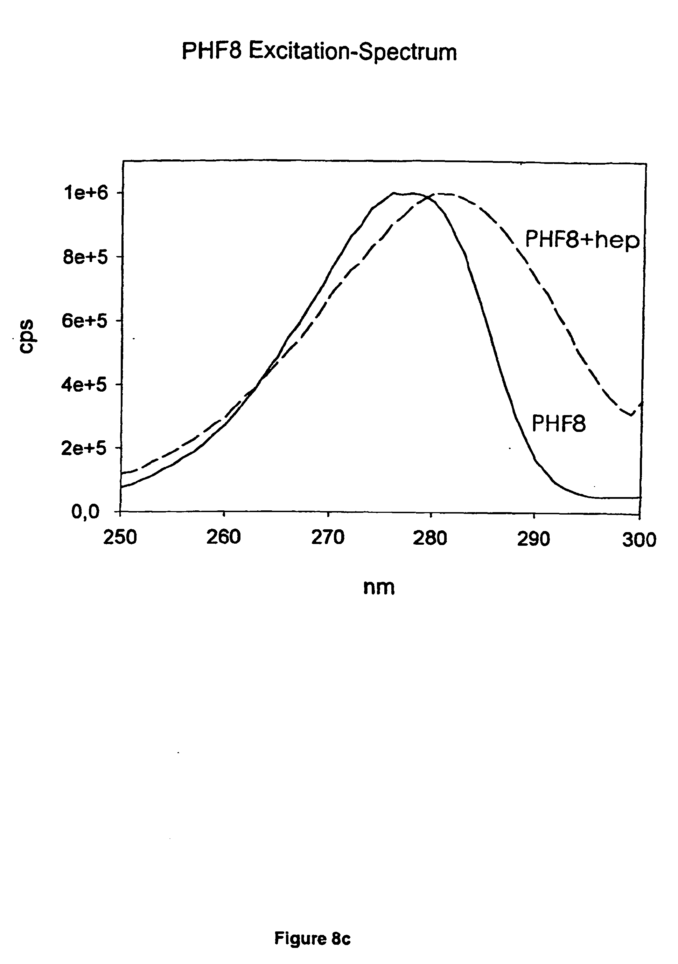

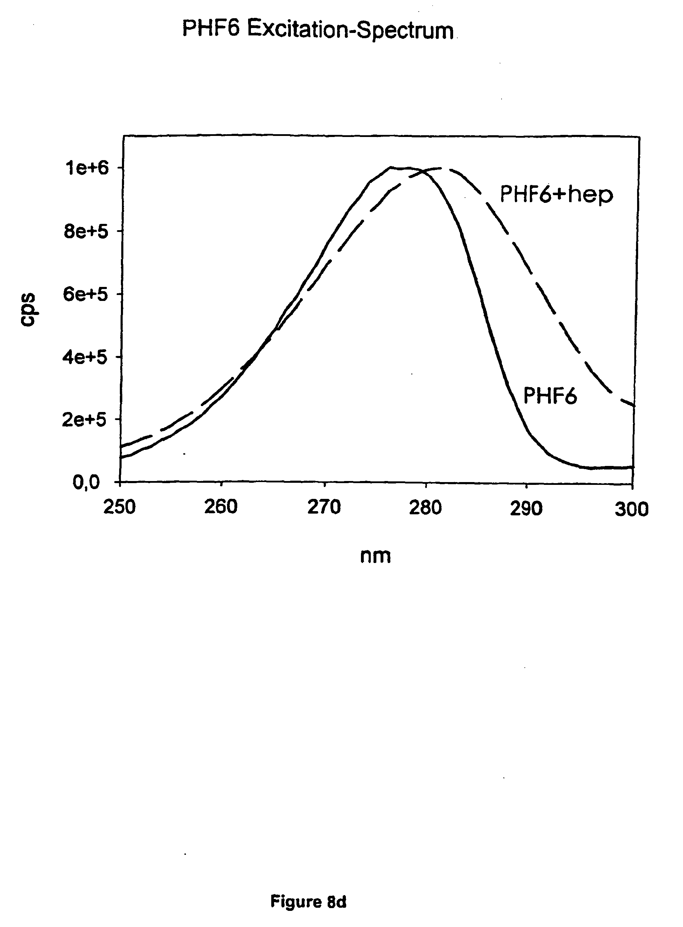

**Fig. 8: Excitation spectra of tyrosine fluorescence of peptides derived from tau protein.** (a) K19, (b) PHF43, (c) PHF8, (d) PHF6. All peptides contain Tyr310 as the only tyrosine. The fluorescence emission is observed at 310 nm, the excitation wavelength is scanned between 250 and 300 nm. Solid lines represent soluble peptides without heparin, dashed lines show peptides after aggregation in the presence of heparin. Note that aggregation causes a shift of the excitation maximum by about 5nm.

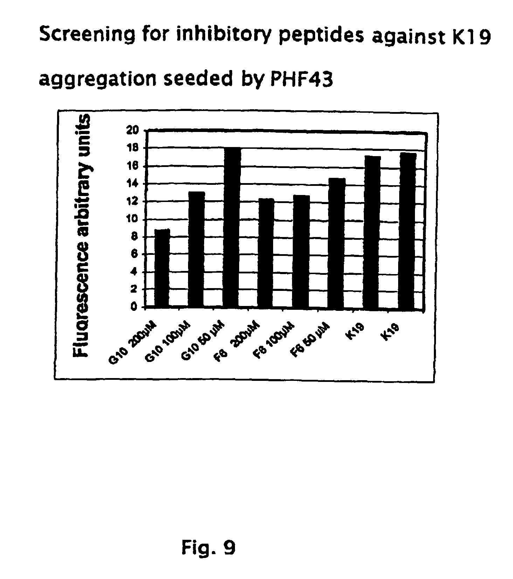

**Fig. 9: Screening for inhibitory peptides.** The peptides were preincubated for 30 minutes at room temperature with K19 (final concentration 50 µM) which PHF aggregation was induced with PHF43 at concentrations of 50, 100 and 200 µM in a 384 well plate and measured by ThS-assay. The inhibitory peptides G10 and F6 were found as interaction partners for the hexapeptide SEQ ID NO: 7 with a phage display approach described above. As a control two samples of K19 without peptides are also shown. Note that the inhibitory peptide G10 at a concentration of 200 µM shows a more than 50 % inhibition of PHF aggregation and the peptide F6 shows a 30 % inhibition at 100 µM.

The invention will now be described by reference to the following biological examples which are merely illustrative and are not to be construed as a limitation of scope of the present invention.

### EXAMPLE I

### Proteolytic degradation products of the repeat domain cause efficient assembly of PHFs

Earlier efforts to reconstitute PHFs in vitro had shown that tau constructs containing roughly the repeat domain polymerized much more readily than the full-length protein (Wille, loc. cit.), consistent with the observation that the repeat domain forms the core of PHFs from Alzheimer brains (Wischik, loc. cit.). To achieve PHF assembly from intact tau it was necessary to add polyanionic cofactors such as heparin, RNA, or poly-Glu (Goedert, loc. cit.; Kampers, loc. cit.; Perez, loc. cit.). However, it was noted that different preparations of tau protein showed a considerable variability in their assembly efficiency, and that some of the more readily aggregating preparations contained proteolytic breakdown products, as judged by SDS gels. Tau constructs were digested with different proteases, the breakdown products were characterized and they were analyzed for their assembly behaviour. In this study, assembly behaviours were studied as follows: varying concentrations of tau isoforms or tau constructs and fragments (typically in the range of 1-100 µM) in volumes of 20-500 µl were incubated at 37°C in 50 mM NH₄Ac, pH 7.0 or 20 mM MOPS-NaOH, pH 7.0 containing anionic cofactors (polyglutamate, RNA or heparin) as described (Friedhoff, loc. cit.). Incubation times varied between minutes up to several days. PHF formation was always confirmed by electron microscopy. Here, construct K19, a derivative of the fetal tau isoform (htau23) was employed for assembly studies. K19 contains only three repeats (R1, R₃, R4; the second repeat R2 is absent due to alternative splicing; Fig. 1). Construct K19 (SEQ ID NO: 10) was expressed, like human tau 23, in E.coli as described (Biernat et al, EMBO J. 11 (1992), 1593-1597). The proteins were expressed and purified as described in the art, employing heat stability and FPLC Mono S (Pharmacia) chromatography, as described, inter alia, in Gustke (1994), Biochemistry 33, 9511-9522. The purity of proteins was verified by SDS-PAGE. Protein concentrations were determined by Bradford assay. Synthetic peptides were obtained from Eurogentec SA (Seraing, Belgium). Construct K19 was digested with chymotrypsin, trypsin, AspN, and GluC. Briefly, different fragments of K19 were generated by proteolysis with the above mentioned proteases. Extensive proteolysis by trypsin (ratio w/w 1:100, in 50 mM NH₄CO₃ pH 8.4, 37°C for 2 h), limited proteolysis by chymotrypsin (ratio w/w 1:200, in 50 mM NH₄CO₃ pH 8.4, RT, for 30 min) and extensive proteolysis by GluC (w/w 1:100, in 50 mM NH₄Ac pH 4.0 or 50 mM NaHPO₄ pH 7.8, RT, ovemight). The specificity of GluC depends on the buffer and pH: in ammonium acetate pH 4.0 only peptide bonds C-terminal of Glu are cleaved (leading to fragments as shown in SEQ ID NOS: 52, 6, 53 and 54), in phosphate buffer pH 7.8 peptide bonds C-terminal of Asp and Glu are cleaved; leading to fragments as shown in SEQ ID NOS: 55 to 59).

Obtained peptides were purified by reverse phase HPLC on a C18 reverse phase column (Vydac) on a SMART HPLC system (Pharmacia) with a standard gradient from 0-50 % buffer B (80% acetonitrile v/v in 10 mM NH₄Ac pH 7.0, buffer A 10 mM NH₄Ac pH 7.0) in 8 ml and 50-100% B in 2 ml. The peptide PHF43 was purified by gel filtration on a Peptide-Gelfiltration (Pharmacia) with 100 mM NH₄Ac pH 7.0 at a flow rate of 40 µl/min. Peptides were lyophilized after HPLC and analyzed by MALDI mass spectrometry (Maldi II, Shimadzu) and N-terminal sequencing (ABI-476). Peptide concentrations were determined by fluorescamine assay (Udenfriend, Science 178 (1972), 871-872).

One of the proteolytic products was a fragment, generated by GluC digestion and termed PHF43 (SEQ ID NO: 6), comprising only 43 residues, essentially the third repeat plus flanking stretches on either site (tau sequence 265-338 without the second repeat, 275-305; the numbering of the amino acids is that of the isoform htau 40 containing 441 residues described in Goedert (1989), EMBO J. 8, 393-399; Figs. 2 and 3). This fragment assembled much more rapidly than other fragments or intact tau, as judged by electron microscopy and the ThS fluorescence assay (half time 0.75 min, compared with 12 min for K19 and 180 min for htau23, Fig. 2). For electron microscopy studies, protein solutions were diluted to 1-10 µM and were placed on 600-mesh carbon-coated copper grids for 1 min and negatively stained with 2% uranyl acetate for 45 sec. The specimen were examined in a Philips CM12 electron microscope at 100 kV. The Thioflavin S-assay was carried out as previously described (Friedhoff et al, loc. cit.). Fluorescence was measured with a Fluoroskan Ascent (Labsytems) with an excitation filter of 480 ± 5±nm and an emission filter of 510 nm ±5 nm in a 384 well plate. Measurements were carried out at room temperature in ammonium acetate 50 mM, pH 7.0 or 20 mM Na-MOPS pH 7.0 with 5 µM ThS unless otherwise stated. Background fluorescence and light scattering of the sample without thioflavine was subtracted when needed.

As with other tau constructs, efficient assembly required the presence of polyanions (e.g. heparin, RNA, or poly-Glu), as well as initial dimerization by oxidation of Cys322 into disulfide bridges, indicating that dimers form the effective building blocks of assembly (Friedhoff, loc. cit.; Schweers, loc. cit.). In fact PHF43 showed an unusually strong tendency to dimerize (in the absence of DTT) so that more than half was already dimerized upon digestion of K19, and after elution from the reverse phase column the dimer fraction was close to 100%. The reaction was based on the crosslinking of Cys322, the only sulfhydryl group in K19 or PHF43, and could be reversed by reducing agents (e.g. DTT).

### EXAMPLE II

### PHF43 alone forms straight filaments but rapidly nucleates bona fide PHFs from tau23

Electron microscopy was carried out as described herein above. It could be shown that most fibers obtained after self-assembly of the peptide PHF43 (see Example I) appeared as straight thin filaments, often aggregated laterally, and lacking the periodic -80 nm supertwist that is characteristic of Alzheimer PHFs, which is in contrast to fibers assembled from construct K19 or tau23. Since PHF assembly follows a nucleation-condensation mechanism (Friedhoff, Proc. Natl. Acad. Sci. USA (1998b), 15712-15717), fragments of PHF43 fibers were prepared by sonication (sonicator: Branson scientific; 2 min; output level 5; duty cycle 50%; as described in Friedhoff (1998b), loc.cit.) and it was asked whether these could function as seeds for PHF assembly from larger tau constructs or full-length htau23. Surprisingly, the seeds made from the fragmented PHF43 filaments were capable of rapidly nucleating bona fide PHFs from K19 or htau23, displaying the typical 80 nm twist (Fig. 2, 4). This means that the interaction between PHF43 molecules must be very similar to that of PHFs, both in a kinetic sense (requiring dimerization and polyanions) and in a structural sense (nucleation of twisted fibers). The smaller diameter of PHF43-fibers could be accounted for by the small size of the peptide, compared to the larger tau constructs and isoforms studied previously (Kampers, loc. cit.; Wille, loc. cit.).

Therefore, PHF43, representing the third repeat plus short flanking sequences of tau assembles within seconds into thin fibers under the same conditions as full-length tau (favored by dimerization and anionic cofactors such as heparin, Fig. 2). Seeds derived by sonication from such fibers are capable of greatly accelerating the assembly of full-length tau into bona fide PHFs, showing that the PHF43 interactions are equivalent to the interactions in PHFs (Fig. 4). Interestingly, K19 mutants, which comprise modifications located within the hexapeptide PHF6 (see herein below), showed no aggregation even after several hours (filled triangle at the bottom right of Fig. 2). These K19 mutations are: K19^{ΔQI}, K19^{NAE} and K19^{V310E} as indicated in Fig. 3 (see also SEQ ID Nos: 12 to 14).

### EXAMPLE III

### The hexapeptide PHF6 (306-VQIVYK-311, SEQ ID NO: 8) represents a minimal interaction motif with a predicted beta conformation

In order to test whether the tau-tau interaction can be traced down to an even smaller motif a membrane containing spots of immobilized 15-mer peptides, covering the sequence of the repeats with overlaps of 3 residues between successive peptides (Frank, loc. cit.).was used. This spot membrane binding assay was carried out as follows: lodo-beads (Pierce) were preincubated with Na¹²⁵I (0.5 mCi in 100 mM Tris pH 7.0) for 5 minutes at room temperature. The purified peptide PHF43 (50 µg) was iodinated at Y310 for 5 minutes at room temperature. The reaction was stopped by removing the solution and the mixture was applied to a 1 ml C18 cartridge (Waters), equilibrated with 0.1 % TFA. The unbound radioactivity was washed out with 4 ml TFA (0.1 %). The radioactive peptide was eluted from the column with 66% acetonitrile, 0.1 % TFA. All fractions were analyzed by scintillation counting. The eluate of the labeled peptide was lyophilized and diluted in ammonium acetate 50 mM. The membrane containing the library of overlapping peptides was synthesized according to Frank (1995; loc. cit.). Before use, the membrane was incubated in 100% ethanol, then washed three times with tris buffered saline (5 min, room temperature). The labeled peptide solution was diluted 10-fold in TBS (final concentration 0.1 mg/ml). The membrane was incubated in the peptide solution for 2 hours at room temperature, then washed three times with TBS. Autoradiography on Kodak X-omat film was done overnight.

The peptide spot membrane was probed against the PHF43 peptide, radioactively labeled by iodination with ¹²⁵I at Tyr310. The most prominent interaction between PHF43 and the spot membrane was found at peptides surrounding Tyr310 (Fig. 5). This means that the region around residue 310 tends to dimerize. A motif overlapping with the interacting peptides was the hexapeptide ³⁰⁶VQIVYK³¹¹ (termed PHF6, bold in Fig. 5, SEQ ID NO: 8), at the beginning of R3. A second maximum of interaction was centered around Ile278, covering the sequence ²⁷⁵VQIINK²⁸⁰ (SEQ ID NO: 7). This sequence is the equivalent of PHF6 at the beginning of R2 (present only in 4-repeat tau and included in the spot membrane). Remarkably, PHF6 in R3 and its counterpart in R2 represent the only regions in tau where a beta conformation is strongly predicted, consistent with the local clustering of residues with high β-sheet propensity such as V, I, Q, and Y. The PHF-forming interaction between tau or tau-derived peptides is strongly dominated by the hexapeptide ³⁰⁶VQIVYK³¹¹ (PHF6), derived from the third repeat R3, or the equivalent sequence ²⁷⁵VQIINK²⁸⁰ (now termed PHF6*) from the second repeat R2 (Fig. 5).

### EXAMPLE IV

### CD spectroscopy of tau fragments suggests a β-structure in PHF assembly conditions

The conformation of proteins can be probed by circular dichroism, infrared spectroscopy, or solution X-ray scattering. Earlier studies (Schweers (1995), loc.cit.; Wille (1992), loc.cit.) of tau isoforms had shown that the ordered secondary structure of tau (α-helix or β-sheet) was minimal, below the reliable detection limit of the methods (~5-10%). This had given rise to the notion that tau was a largely random structure, without a well-defined folding pattern, reminiscent of a Gaussian polymer (Schweers (1994), loc. cit.). This view did however not exclude the possibility of a locally ordered structure which should become more visible with shorter tau-derived peptides. Therefore, the CD spectra of tau and its fragments in several conditions (Fig. 6 a-f and Fig. 7) were studied. All measurements were made with a Jasco J-710 CD-Dichrograph (Jasco, Japan) in a cuvette with 0.05 cm path length. For each experiment, 10 spectra were summed up and the molar ellipticity was determined after normalizing for the protein concentrations. The secondary structure interpretation of the CD-data was performed according to Chou and Fasman (Annu. Rev. Biochem. 47 (1978), 251-276), as implemented in the program Dicroprot (Deleage and Geourjon, Comput Appl Biosci, 9 (1993), 197-9).

The above mentioned conditions for CD spectra experiments of tau and its fragments and/or corresponding constructs were as follows:
- (a) The monomeric state: This is obtained in the presence of DTT (1mM) because this keeps Cys322 reduced and prevents dimer formation.
- (b) The dimeric state is obtained by allowing air oxidation of Cys322; this results in almost complete conversion from monomers to dimers (see Schweers, loc. cit.). Dimers of tau isoforms or tau constructs were allowed to form by incubation at 10 mg /ml after removal of DTT. The dimers were separated from monomers by gel filtration on a Superdex^{™} 75 column (Pharmacia) equilibrated with 200 mM NH₄Ac, pH 7.0. Fractions were collected and assayed for dimeric K19 by 15 % SDS-PAGE under non-reducing conditions.
- (c) Monomers with added polyanions, e.g. heparin: This could reveal a conformational change induced by heparin independently of aggregation since PHF assembly is normally inefficient in the absence of dimers. Heparin (average MW of 6000) was obtained from Sigma. For CD experiments as described herein, only heparin was usable as cofactor for assembly because poly-Glu interferes with the CD signal of tau and RNA has a strong absorption in the UV range.
- (d) Dimers with added polyanions: This conditions causes the aggregation of tau into PHFs. Concomitant conformation changes can be observed by comparing the initial solution and the final aggregated state.
- (e) Monomers, dimers, or aggregates in the presence of TFE (trifluoroethanol; see Fig. 5), a strong inducer of alpha-helical conformation: This should convert those fractions of the protein that are free to change their conformation into α-helix.

CD spectra are often interpreted in terms of a few idealized structures and their reference spectra (alpha-helix, beta-sheet, random coil, turns). The method yields approximate secondary structure fractions for well-folded proteins but has wide error margins in the case of "natively unfolded" proteins such as tau and interacting cofactors. The spectra were formally interpreted following the method of Chou & Fasman (loc. cit.) and expressed as percentage of alpha, beta, and random coil (Fig. 7). More significant are however the general shapes of the spectra which reveal gross conformational changes. All curves obtained for htau23 have negative molar ellipticities at wavelengths between 190 and 200 nm and show a minimum around 200 nm. This is characteristic of mostly random coil structures and confirms our earlier observations (Schweers (1994), loc. cit.). Neither dimerization nor heparin changes the spectra substantially, arguing that the random coil structure dominates in all cases, even after PHF assembly (solid curve in Fig. 6a). Even if a local beta-structure were formed during PHF assembly, this does not become noticeable with full-length tau. The situation changes as the tau constructs become smaller (Fig. 6b): K19 also displays a mostly random coil structure in the monomeric or dimeric state, even with heparin, but polymerization induces a noticeable change in the spectra so that the minimum becomes wider and is shifted towards higher wavelengths, indicating a substantial change from random coil to beta structure. This behavior is reiterated in the case of PHF43 (Fig. 6c), showing mostly random coil structures, except after PHF assembly where the content of beta-structure is increased. Fig. 6d compares the curves of PHF6 (VQIVYK) before and after assembly through addition of heparin, as well as PHF8 (= PHF6 plus the preceding two residues, GKVQIVYK, SEQ ID NO: 9); again there is a substantial change from random coil to beta structure after assembly. Note that in the latter two cases the intermolecular interaction is not preceded by disulfide oxidation since Cys322 is not present. Next several peptides derived from the repeat domain of tau were probed. The nonapeptide ³¹³VDLSKVTSK³²¹ (SEQ ID NO: 14) from R3 following after PHF6 does not polymerize and shows mostly random coil with some beta-structure in the presence or absence of heparin. The same is true for the 18-mer peptide ³¹⁸VTSKCGSLGNIHHKPGGG³³⁵ (SEQ ID NO: 15) which represents the second half of R3 that is more conserved between the repeats (sometimes termed the "repeat" in the strict sense, peptide provided by R. Hoffmann and L. Otvos, see Hoffmann, J. Pept. Res. 50 (1997), 132-142). Note that this peptide does not assemble even though it contains Cys322, indicating that the vicinity of Cys alone is not sufficient for PHF formation. Finally, the peptide ³³⁵GQVEVKSE³⁴² (SEQ ID NO: 16) shows a similar behavior, i.e. mostly random coil with some beta-structure, with or without heparin, and no assembly. This peptide is located at the beginning of R4, analogously to PHF8, but evidently does not have the capacity to interact with itself, presumably because it contains sheet-breaking residues. (Note that for the non-assembling peptides the assignment of beta-structure cannot be taken literally since no beta-sheets are formed. The numbers are merely generated by the computer algorithm whose theoretical assumptions are only partly fulfilled here).

In a further set of experiments the influence of the helix inducer TFE (50%) on the CD spectra was tested. The rationale was that monomeric or dimeric molecules should show a substantial conversion to helical structure, whereas polymerized fibers should be less convertible because part of their sequence was locked in the PHF-forming conformation. This expectation was broadly supported by the data (Fig. 6f): In all cases tested, TFE induced a strong shift towards a more helical spectra, as seen by the positive molar ellipticities below 200 nm and the broad minimum between 210 and 230 nm. The calculated fraction of alpha-helix was larger for the unpolymerized state than after polymerization, as expected (see Fig. 7). The convertible fraction was increased with the size of the protein (i.e. smallest for PHF43, largest for htau23 consistent with the assumption that only a minor fraction of the protein was locked in the PHF interactions. (Note however that the curves give only a qualitative picture of the conformation but do not allow quantitation of the protein domains actually involved in the intra-filament backbone).

In summary, PHF assembly is accompanied by a substantial increase in β-sheet formation (Fig. 6).

This is not observed upon dimerization or addition of anionic cofactors alone, indicating that beta-sheet formation is an essential part of PHF assembly. (5) The hexapeptides ³⁰⁶VQIVYK³¹¹ in R3 and its counterpart ²⁷⁵VQIINK²⁸⁰ in R2 are the only two spots in the tau sequence where a beta-conformation is strongly predicted. This prediction is based on a comparison of all known sequences of the tau/MAP2/MAP4 family of proteins, searching for a signature motif of this class of MAPs and the corresponding secondary structure (Fig. 7). The pronounced tendency for beta-structure is explained by the local clustering of strong beta-sheet inducing residues such as V, I, Y, and Q, whereas the corresponding hexapeptides in the other repeats R1 (QTAPVPM; SEQ ID NO: 20) and R4 (VEVKSE; SEQ ID NO:21) contain beta-sheet breakers such as P and E.

These observations show that the residues of tau centered around PHF6 that make up PHF43 form the backbone of PHF assembly by a limited beta-sheet interaction, while other sequences of tau remain more peripheral and retain a mostly random-coil structure. PHF43 itself forms only slender fibers, but larger constructs (e.g. K19) or full-length tau show the typical PHF-like twisted appearance; in each case the kernel of the interaction is centered around the PHF6 sequence. Without being bound by theory, several conclusions can be drawn:
(1) The sequence of PHF6 lies at the beginning of R3, just behind exon 10. It is therefore present in all tau isoforms and provides an explanation why all tau isoforms can form PHFs. The counterpart PHF6* lies at the beginning of R2 and is therefore present only in 4-repeat isoforms. If the two hotspots for β-sheet formation operated in a cooperative fashion they could possibly enhance PHF assembly, and this might explain why PHF formation is enhanced in dementias where the 4-repeat isoforms are overrepresented (e.g. FTDP-17, see Clark *et al.,* 1998; Hutton *et al.,* 1998; Spillantini *et al.,* 1998). For example, PHF6 and PHF6* could form an intra-molecular sheet-like interaction, ready to be propagated to neighboring tau molecules. This would imply a local hairpin fold, with a bend possibly at the flexible PGGG motif at the end of R2. The occurrence of such a fold has been suggested by previous fluorescence transfer experiments involving the cysteines in repeats R2 and R3 (Schweers et al., 1995).
(2) Dimerization of tau via oxidation of Cys322 into an inter-molecular disulfide bridge strongly promotes PHF assembly (Wille *et al.,* 1992). In 4R tau there are two cysteines (Cys291 in R2 and Cys322 in R3) which can either form inter-molecular bridges (Cys322-Cys322) which favors PHF assembly, or intra-molecular bridges (Cys291-C322) which locks tau in a folded conformation that is.unfavorable for PHF assembly (Schweers *et al.,* 1995). Independently of that, the results shown here argue that the peptide containing Cys322 (peptide 318-335, Tab. 2) does not promote PHF assembly by itself. However, dimerization at Cys322 could bring two PHF6-motifs into close vicinity which would facilitate beta-sheet interactions. In this view, dimerization would act as an effective enhancer of local tau concentration. The close neighborhood of Cys322 and the PHF6 motif suggests that the molecules would form a parallel beta-sheet.
(3) The role of heparin or other anionic cofactors remains unknown in detail, but could effectively help reduce the repulsion between the cationic tau protein in a general way, as discussed by several authors previously (Friedhoff loc. cit., 1998a; Friedhoff loc. cit., 1998b; Goedert loc. cit., 1996; Hasegawa, J. Biol. Chem. 272 (1997), 33118-33124; Kampers, loc. cit.; Perez, loc. cit.). This would also be equivalent to enhancing the effective local concentration of tau. Heparin does not appear to change the conformation of tau by itself, as judged by the CD spectra, because a shift from random-coil to beta-structure is only observed as polymerization progresses.
(4) In a previous study (Schweers, loc. cit.) it was described that tau is a natively unfolded protein with very little detectable secondary structure (as judged by CD, FTIR, or X-ray scattering). This conclusion still holds true (see Fig. 6a), but needs to be interpreted in more detail: The interaction leading to PHFs is based on a β-sheet structure; however, this encompasses only a small fraction of the whole sequence and therefore is not detectable by spectroscopic or scattering techniques in the full-length protein.
   The presence of a β-sheet interaction also removes a previous dilemma: How could a disordered protein give rise to an ordered filament? The answer is that only a kernel of the structure needs to be ordered. The observations also explain why only the repeat domain of tau is important for PHF assembly, while the N- or C-terminal tails protrude as "fuzzy coat" (Wischik *et al.,* 1988a).
(5) In Alzheimer's disease and related tauopathies, tau is both abnormally phosphorylated and aggregated, and since phosphorylation appears to be the earlier event it was thought that phosphorylation primes tau for aggregation. However, we have shown recently that phosphorylation is antagonistic to PHF assembly, particularly the phosphorylation sites at the 3 or 4 KXGS motifs in the repeats (Ser262, 293, 324, 356, Schneider *et al.,* 1999). It is not known whether these sites interact with the PHF6 motif; but at least Ser324 is very close to the dimer-forming residue Cys322. Consistent with this, phosphorylation at KXGS motifs inhibits dimerization and thus the subsequent assembly via the PHF6 motif, in contrast to other phosphorylation sites which allow dimerization but inhibit PHF assembly at a later stage (e.g. Ser214).
(6) Tau mutations have recently been discovered for several familial frontotemporal dementias (Clark *et al.,* 1998; Hutton *et al.,* 1998; Poorkaj *et al.,* 1998; Spillantini et *al*., 1998). Some of them are intronic mutations that affect the splicing of tau (upregulation of 4R tau); the exonic mutations cause changes or loss of residues in the repeat domain of tau. It is notable that several of these lie close to the motifs of PHF6 (e.g. G272V in 3R-tau, P301 L in 4R-tau, see Fig. 1), PHF6* (e.g. G272V in 4R-tau, N279K, ΔK280), or in an analogous position at the beginning of R4 (e.g. V337M) and could possibly enhance the ability of the mutant tau to aggregate.

### EXAMPLE V

### Aggregation assay of tau and tau-derived peptides and detection by tyrosine self-fluorescence (Tyrosin-Assay)

This assay is applicable particularly to the aggregation of small peptides (e.g. PHF6 as shown in SEQ ID NO: 8; K19-fragments as depicted, inter alia, in SEQ ID NO:57 or 6; K19 as shown in SEQ ID NO: 10; PHF43 as depicted in SEQ ID NO:6; PHF8 as shown in SEQ ID NO:9). It can be performed in a cuvette or a 384 well plate. The assay is aplicable to all tau isoforms and fragments containing Tyr310, but is particularly suitable for constructs derived from the repeat domain which contain only this one tyrosine 310, e.g. K19 (Fig. 8a), PHF43 (Fig. 8b), PHF8 (Fig. 8c) or PHF6 (Fig. 8d). This assay is based on the fact that the environment of Tyr310 changes during aggregation. As a result of the aggregation the excitation maximum of the intrinsic tyrosine fluorescence (observed at the emission maximum at 310 nm) shows a red-shift by about 5nm (e.g. from 278 to 283 nm).

Reagents for 50 µl reaction volume:
17.5 µl H₂O
5 µl buffer NH₄Ac 200 mM, pH 7.0 (final concentration 20 mM)
25 µl heparin MW 3000, 1 mM (final concentration 500 µM)
2.5 µl peptide (e.g. K19, PHF43, PH19, PH8, or PHF6), 10 mM (final concentration 500 µM)

The reaction volume is placed in a fluorimeter (e.g. Fluoromax, ISA GmbH). The increase in aggregation can be measured by observing the excitation spectrum between 250 and 300 nm (Fig. 8a-d; figures show the excitation spectra before and after aggregation) at a fixed emission wavelength (310 nm).

### EXAMPLE VI

### Aggregation analysis of full-length-tau (isoform htau 23), fragments thereof comprising the repeat domain, PHF43 and PHF6

Human full-length tau23 is expressed in E. coli, purified as described (e.g. Friedhoff, 1998a loc. cit.), and stored in 10 mM NH₄ -acetate buffer pH 7.0. The aggregation experiment is set up as follows (for 50 µl reaction volume):
35 µl H₂O
5 µl buffer NH₄-acetate 200 mM, pH 7.0 (final concentration 20 mM)
5 µl heparin MW 3000, 500 µM (final concentration 50 µM)
5 µl tau protein 500 µM (final concentration 50 µM)

The solution is incubated at room temperature for 12 hours, during which time the protein aggregates.

The detection of aggregation by fluorescence is set up as follows (for 50 µl reaction volume):
34 µl H₂O
5 µl buffer NH₄-acetate, 200 mM, pH 7.0;
1 µl Thioflavine S, 100 µM (final concentration 2 µM),
10 µl tau protein from aggregation reaction (see above), 50 µM (final concentration 5 µM).

The solution is incubated for 10 min, placed in a cuvette in a fluorimeter (Fluoroskan Ascent, Labsystems) at room temperature. The fluorescence of Thioflavine S bound to the aggregated protein is determined at an excitation wavelength of 440 nm and an emission wavelength of 510 nm. The degree of aggregation is determined by reference with a previously determined calibration series. As an additional control the presence of aggregated protein is observed by negative stain electron microscopy.

Human tau fragment K19 is expressed in E. coli, purified as described (e.g. Friedhoff et al., 1998a), and stored in 10 mM NH₄ -acetate buffer pH 7.0. The aggregation experiment is set up as follows (for 50 µl reaction volume):
35 µl H₂O
5 µl buffer NH₄-acetate 200 mM, pH 7.0 (final concentration 20 mM)
5 µl heparin MW 3000, 500 µM (final concentration 50 µM)
5 µl K19 protein 500 µM (final concentration 50 µM)

The solution is incubated at room temperature for 1 hour, during which time the protein aggregates.

The detection of aggregation by fluorescence is set up as follows (for 50 µl reaction volume):
34 µl H₂O
5 µl buffer NH₄-acetate, 200 mM, pH 7.0;
1 µl Thioflavine S, 100 µM (final concentration 2 µM),
10 µl K19 protein from aggregation reaction (see above), 50 µM (final concentration 5 µM).

The solution is incubated for.10 min, placed in a cuvette in a fluorimeter (Fluoroskan Ascent, Labsystems) at room temperature. The fluorescence of Thioflavine S bound to the aggregated protein is determined at an excitation wavelength of 440 nm and an emission wavelength of 510 nm (see examples herein above). The degree of aggregation is determined by reference with a previously determined calibration series. As an additional control the presence of aggregated protein is observed by negative stain electron microscopy.

Human tau fragment PHF43 (SEQ ID NO: 6) is expressed in E. coli, purified as described above, and stored in 10 mM NH₄ -acetate buffer pH 7.0. The aggregation experiment is set up as follows (for 50 µl reaction volume):
35 µl H₂O
5 µl buffer NH₄-acetate 200 mM, pH 7.0 (final concentration 20 mM)
5 µl heparin MW 3000, 500 µM (final concentration 50 µM)
5 µl PHF43 protein 500 µM (final concentration 50 µM)

The solution is incubated at room temperature for 4 min, during which time the protein aggregates.

The detection of aggregation by fluorescence is set up as follows (for 50 µl reaction volume):
34 µl H₂O
5 µl buffer NH₄-acetate, 200 mM, pH 7.0;
1 µl Thioflavine S, 100 µM (final concentration 2 µM),
10 µl PHF43 from aggregation reaction (see above), 50 µM (final concentration 5 µM).

The solution is incubated for 10 min, placed in a cuvette in a fluorimeter (Fluoroskan Ascent, Labsystems) at room temperature. The fluorescence of Thioflavine S bound to the aggregated protein is determined at an excitation wavelength of 440 nm and an emission wavelength of 510 nm. The degree of aggregation is determined by reference with a previously determined calibration series. As an additional control the presence of aggregated protein is observed by negative stain electron microscopy.

Peptide PHF6 (SEQ ID NO: 8) is synthesized by standard peptide synthesis and dissolved in 10 mM NH₄ -acetate buffer pH 7.0. The aggregation experiment is set up as follows (for 50 µl reaction volume):
17.5 µl H₂O
5 µl buffer NH₄-acetate 200 mM, pH 7.0 (final concentration 20 mM)
25 µl heparin MW 3000, 1 mM (final concentration 500 µM)
2.5 µl PHF6 peptide 10 mM (final concentration 500 µM)

The peptide aggregates within seconds; the solution is diluted 100-fold and placed immediately into a cuvette in a fluorimeter (Fluoromax, ISA GmbH). The intrinsic tyrosine fluorescence of the peptide PHF6 is determined at an excitation wavelength of 280 nm and an emission wavelength of 310 nm. The degree of aggregation is determined by reference with a previously determined calibration series. As an additional control the presence of aggregated protein is observed by negative stain electron microscopy. As shown herein above, wherein assays based on full-length tau or fragments comprising the repeat domain of tau require in vitro aggregation times of at least one hour (K19 see above) or even 12 hours (full length tau, see above), assays based on PHF43 (SEQ ID NO: 6) or PHF6 (inter alia, SEQ ID NO: 8) need minimal primary incubation times of 4 min (see above in the case of PHF43) or even only seconds (see PHF6).

### EXAMPLE VII

### Automated screening of potential inhibitors of PHF assembly

Human tau fragment PHF43, purified as describe above and stored in 10 mM NH4-actetate buffer pH 7.0. The aggregation experiment is set up as folllows (for 50 µl reaction volume):
35 µl H₂O
5 µl buffer NH₄-acetate 200 mM, pH 7.0 final concentration 20 mM)
5 µl heparin MW 3000, 500 µM (final concentration 50 µM)
5 µl PHF43 protein 500 µM (final concentration 50 µM)

The solution is incubated at room temperature for 1 hour, during which time the protein aggregates.

The polymerization reaction of human tau fragment PHF43 is performed in a well of a 384 well plate (Nunc).

The detection of aggregation by fluorescence is set up as follows (for 50 µl reaction volume):
34 µl H₂O
5 µl buffer NH₄-acetate, 200 mM, pH 7.0;
1 µl Thioflavine S, 100 µM (final concentration 2 µM),
10 µl PHF43 protein from aggregation reaction (see above), 50 µM (final concentration 5 µM).

For detection the samples are transferred from the incubation plate to another 384 well plate (Labsystem) with the following conditions. This 384 well plate (Labsystem) is used for measuring the ThS fluorescence with the said excitation and emission wavelengths.

The solution is incubated for 10 min., the 384 well plate is placed in a fluorimeter (Fluoroskan Ascent, Labsystems) at room temperature. The fluorescence of Thioflavine S bound to the aggregated protein is determined at an excitation wavelength of 440 nm and an emission wavelength of 510 nm. The degree fo aggregation is determined by reference with a previously determined calibration series. As an additional control the presence of aggregated protein is observed by negative stain electron microscopy.

### EXAMPLE XIII

### Screening for inhibitors

A typical screening using different peptides derived from a peptide phage display (NEB) using following conditions:
a) Phage display is performed using a M-13 based system (PhD.-7 Peptide 7-mer Library Kit, New England Biosystem) with a randomized heptamer amino acid as fusion seqeunce to a coat protein of the phage. Enrichment of tau binding peptides from this library was performed by coating PHF43 to the surface of 96 well plates (Maxisorb, Nunc), incubation with the library and after several times of washing eluting by a 0.1 mg/ml solution of Hexappetide VQIVYK in PBS. The eluted phages were grown up and reamplified for additional rounds of panning. After four rounds of panning phages were plated and individual clones were sequenced according to the recommendations of the supplier. Peptides of interest were synthesized by the company Eurogentech and used for the screening for inhibitory peptides for PHF formation of PHF43 and tau derived peptides seeded with PHF43 (Fig.9).
   Two inhibitory peptides against the Hexapeptides PHF6 (SEQ ID NO: 7) were found. G10 and F6, which inhibit PHF formation at 200 µM to 50 % and 30 %, respectively.
b) The polymerization reaction of human tau fragment PHF43 is performed in a well of a 384 well plate (Nunc).

### 1. PHF43 final concentration 50 µM, peptide final concentration 50 µM

Preincubation of peptides to PHF43 30 min. at room temperature:
32.5 µL H20
5 µl PHF43, 500 µM (final concentration 50 mM)
2.5 µl Peptide 1 mM (final concentration 50 µM)
5 µl NH₄-Ac 100 mM, pH 7.0

After 30 minutes 5µl of heparin MW 3000, 500 µM (final concentration 50 µl) are added. The solution is incubated at room temperature for 1 hour, during which time the protein aggregates.

The detection of aggregation by fluorescence is set up as follows (for 50 µl reaction volume)
34 µl H₂O
5 µl buffer NH₄-Ac 200 mM, pH 7.0
1 µl Thioflavine S, 100 µM (final concentration 2 µM)
10 µl PHF protein from aggregation reaction (see above), 50 µM (final concentration 5 µl)

For detection the samples are transferred from the incubation plate to another 384 well plate (Labsystem). The 384 well plate (Labsystem) is used for measuring the ThS fluorescence with the said excitation and emission wavelengths.

The solution is incubated for 10 min., the 384 well plate is placed in a fluroimeter (Fluoroskan Ascent, Labsystems) at room temperature. The fluorescence of Thioflavine S bound to the aggregated protein is determined at an excitation wavelength of 440 nm and an emission wavelength of 510 nm. The degree of aggregation is determined by reference with a previously determined calibration series. As an additional control the presence of aggregated protein is observed by negative stain electron microscopy.

### 2. PHF43 final concentration 50 µM, Peptide final concentration 100 µM

Preincubation of peptides to PHF43 30 min. at room temperature:
30 µl H20
5 µl PHF43 500 µM (final concentration 50 mM)
5 µl Peptid 1 mM (final concentration 100 µM)
5 µl NH₄-Ac 100 mM, pH 7.0

After 30 minutes 5 µl of heparin MW 3000, 500 µM (final concentration 50 µl) are added.

The solution is incubated at room temperature for 1 hour, during which time the protein aggregates.

The detection of aggregation by fluorescence is set up as follows (for 50 µl reaction volume)
34 µl H₂O
5 µl buffer NH₄-Ac 200 mM, pH 7.0
1 µl Thioflavine S, 100 µM (final concentration 2 µM)
10 µl PHF43 protein from aggregation reaction (see above), 50 µM (final concentration 5 µl).

For detection the samples are transferred from the incubation plate to another 384 well plate (Labsystem). This 384 well plate (Labsystem) is used for measuring the ThS fluorescence with the said excitation and emission wavelengths.

The solution is incubated for 10 min., the 384 well plate is placed in a fluorimeter (Fluoroskan Ascent, Labsystems) at room temperature. The fluorescence of Thioflavine S bound to the aggregated protein is determined at an excitation wavelength of 440 nm and an emission wavelength of 510 nm. The degree of aggregation is determined by reference with a previously determined calibration series. As an additional control the presence of aggregated protein is observed by negative stain electron microscopy.

### 3. PHF43 final concentration 50 µM, Peptide final concentration 200 µM

Preincubation of peptides to PHF43 30 min. at room temperature:
25 µl H₂O
5 µl PHF43 500 µM (final concentration 50 mM)
10 µl Peptide 1 mM (final concentration 200 µM)
5µl NH₄-Ac 200 mM, pH 7.0

After 30 minutes 5 µl of heparing MW 3000, 500 µM (final concentration 50 µl) are added.

After 30 minutes 5 µl of heparing MW 3000, 500 µM (final concentration 50 µl) are added. The solution is incubated at room temperature for 1 hour, during which time the protein aggregates.

The detection of aggregation by fluorescence is set up as follows (for 50 µl reaction volume):
34 µl H₂O
5 µl buffer NH₄-Ac 200 mM, pH 7.0
1 µl Thioflavine S, 100 µM (final concentration 2 µM)
10 µl PHF43 protein from aggregation reaction (see above), 50 µM (final concentration 5 µl).

For detection the samples are transferred from the incubation plate to another 384 well plate (Labsystem). This 384 well plate (Labsystem) is used for measuring the ThS fluorescence with the said excitation and emission wavelengths.

The solution is incubated for 10 min., the 384 well plate is placed in a fluorimeter (Fluoroskan Ascent, Labsystems) at room temperature. The fluorescence of Thioflavine S bound to the aggregated protein is determined at an excitation wavelength of 440 nm and an emission wavelength of 510 nm. The degree of aggregation is determined by reference with a previously determined calibration series. As an additional control the presence of aggregated protein is observed by negative stain electron microscopy (Fig. 9).

### EXAMPLE IX

### Seeding by soluble PHF43

Human tau fragment K19 is expressed in E. coli, purified as described (e.g. Friedhoff et al., 1998a), and stored in 10 mM NH₄-acetate buffer pH 7.0. The aggregation experiment is set up as follows (for 50 µl reaction volume):
33,75 µl H₂O
5 µl buffer NH₄-acetate 200 mM, pH 7.0 (final concentration 20 mM)
5 µl heparin MW 3000, 500 µM (final concentration 50 µM)
5 µl K19 protein 500 µM (final concentration 50 µM)
1.25 µl PHF43 (SEQ ID NO:6) 100 µM in 10 mM NH₄-acetate buffer

The solution is incubated at room temperature for 1 hour, during which time the protein aggregates.

The detection of aggregation by fluorescence is set up as follows (for 50 µl reaction volume):
34 µl H₂O
5 µl buffer NH₄-acetate, 200 mM, pH 7.0
1 µl Thioflavine S, 100 µM (final concentration 2 µM),
10 µl K19 protein from aggregation reaction that was seeded with soluble PHF43; see above), 50 µM (final concentration 5 µM).

The solution is incubated for 10 min., placed in a cuvette in a fluorimeter (Fluoroskan Ascent, Labsystems) at room temperature. The fluorescence of Thioflavine S bound to the aggregated protein is determined at an excitation wavelength of 440 nm and an emission wavelength of 510 nm (see examples herein above). The degree of aggregation is determined by reference with a previously determined calibration series. As an additional control the presence of aggregated protein is observed by negative stain electron microscopy. Aggregation of full length tau isoforms as well as peptides derived from tau, described in the literature or disclosed here, as well as FTDP-mutations (Wilhelmsen (1999) loc. cit.) of tau can equally be seeded with soluble PHF43 (SEQ ID NO: 6) and speeded up and analyzed as described above.

### SEQUENCE LISTING

<110> Max-Planck-Gesellschaft zur Förderung der Wissenschaften e.V.
<120> A minimal tau peptide for the nucleation of paired helical filaments
<130> D1894ep
<140>
   <141>
<160> 59
<170> PatentIn Ver. 2.1
<210> 1
   <211> 125
   <212> PRT
   <213> homo sapiens
<400> 1
   Gln Thr Ala Pro Val Pro Met Pro Asp Leu Lys Asn Val Lys Ser Lys
   1 5 10 15
   Ile Gly Ser Thr Glu Asn Leu Lys His Gln Pro Gly Gly Gly Lys Val
   20 25 30
   Gln Ile Ile Asn Lys Lys Leu Asp Leu Ser Asn Val Gln Ser Lys Cys
   35 40 45
   Gly Ser Lys Asp Asn Ile Lys His Val Pro Gly Gly Gly Ser Val Gln
   50 55 60
   Ile Val Tyr Lys Pro Val Asp Leu Ser Lys Val Thr Ser Lys Cys Gly
   65 70 75 80
   Ser Leu Gly Asn Ile His His Lys Pro Gly Gly Gly Gln Val Glu Val
   85 90 95
   Lys Ser Glu Lys Leu Asp Phe Lys Asp Arg Val Gln Ser Lys Ile Gly
   100 105 110
   Ser Leu Asp Asn Ile Thr His Val Pro Gly Gly Gly Asn
   115 120 125
<210> 2
   <211> 31
   <212> PRT
   <213> Homo sapiens
<400> 2
   Gln Thr Ala Pro Val Pro Met Pro Asp Leu Lys Asn Val Lys Ser Lys
   1 5 10 15
   Ile Gly Ser Thr Glu Asn Leu Lys His Gln Pro Gly Gly Gly Lys
   20 25 30
<210> 3
   <211> 31
   <212> PRT
   <213> Homo sapiens
<400> 3
   Val Gln Ile Ile Asn Lys Lys Leu Asp Leu Ser Asn Val Gln Ser Lys
   1 5 10 15
   Cys Gly Ser Lys Asp Asn Ile Lys His Val Pro Gly Gly Gly Ser
   20 25 30
<210> 4
   <211> 31
   <212> PRT
   <213> Homo sapiens
<400> 4
   Val Gln Ile Val Tyr Lys Pro Val Asp Leu Ser Lys Val Thr Ser Lys
   1 5 10 15
   Cys Gly Ser Leu Gly Asn Ile His His Lys Pro Gly Gly Gly Gln
   20 25 30
<210> 5
   <211> 32
   <212> PRT
   <213> Homo sapiens
<400> 5
   Val Glu Val Lys Ser Glu Lys Leu Asp Phe Lys Asp Arg Val Gln Ser
   1 5 10 15
   Lys Ile Gly Ser Leu Asp Asn Ile Thr His Val Pro Gly Gly Gly Asn
   20 25 30
<210> 6
   <211> 43
   <212> PRT
   <213> Homo sapiens
<400> 6
   Asn Leu Lys His Gln Pro Gly Gly Gly Lys Val Gln Ile Val Tyr Lys
   1 5 10 15
   Pro Val Asp Leu Ser Lys Val Thr Ser Lys Cys Gly Ser Leu Gly Asn
   20 25 30
   Ile His His Lys Pro Gly Gly Gly Gln Val Glu
   35 40
<210> 7
   <211> 6
   <212> PRT
   <213> Homo sapiens
<400> 7
   Val Gln Ile Ile Asn Lys
   1 5
<210> 8
   <211> 6
   <212> PRT
   <213> Homo sapiens
<400> 8
   Val Gln Ile Val Tyr Lys
   1 5
<210> 9
   <211> 8
   <212> PRT
   <213> Homo sapiens
<400> 9
   Gly Lys Val Gln Ile Val Tyr Lys
   1 5
<210> 10
   <211> 94
   <212> PRT
   <213> Homo sapiens
<400> 10
   Gln Thr Ala Pro Val Pro Met Pro Asp Leu Lys Asn Val Lys Ser Lys
   1 5 10 15
   Ile Gly Ser Thr Glu Asn Leu Lys His Gln Pro Gly Gly Gly Lys Val
   20 25 30
   Gln Ile Val Tyr Lys Pro Val Asp Leu Ser Lys Val Thr Ser Lys Cys
   35 40 45
   Gly Ser Leu Gly Asn Ile His His Lys Pro Gly Gly Gly Gln Val Glu
   50 55 60
   Val Lys Ser Glu Lys Leu Asp Phe Lys Asp Arg Val Gln Ser Lys Ile
   65 70 75 80
   Gly Ser Leu Asp Asn Ile Thr His Val Pro Gly Gly Gly Asn
   85 90
<210> 11
   <211> 94.
   <212> PRT
   <213> Homo sapiens
<400> 11
   Gln Thr Ala Pro Val Pro Met Pro Asp Leu Lys Asn Val Lys Ser Lys
   1 5 10 15
   Ile Gly Ser Thr Glu Asn Leu Lys His Gln Pro Gly Gly Gly Lys Asn
   20 25 30
   Ala Glu Val Tyr Lys Pro Val Asp Leu Ser Lys Val Thr Ser Lys Cys
   35 40 45
   Gly Ser Leu Gly Asn Ile His His Lys Pro Gly Gly Gly Gln Val Glu
   50 55 60
   Val Lys Ser Glu Lys Leu Asp Phe Lys Asp Arg Val Gln Ser Lys Ile
   65 70 75 80
   Gly Ser Leu Asp Asn Ile Thr His Val Pro Gly Gly Gly Asn
   85 90
<210> 12
   <211> 91
   <212> PRT
   <213> Homo sapiens
<400> 12
   Gln Thr Ala Pro Val Pro Met Pro Asp Leu Lys Asn Val Lys Ser Lys
   1 5 10 15
   Ile Gly Ser Thr Glu Asn Leu Lys His Gln Pro Gly Gly Gly Lys Val
   20 25 30
   Tyr Lys Pro Val Asp Leu Ser Lys Val Thr Ser Lys Cys Gly Ser Leu
   35 40 45
   Gly Asn Ile His His Lys Pro Gly Gly Gly Gln Val Glu Val Lys Ser
   50 55 60
   Glu Lys Leu Asp Phe Lys Asp Arg Val Gln Ser Lys Ile Gly Ser Leu
   65 70 75 80
   Asp Asn Ile Thr His Val Pro Gly Gly Gly Asn
   85 90
<210> 13
   <211> 94
   <212> PRT
   <213> Homo sapiens
<400> 13
   Gln Thr Ala Pro Val Pro Met Pro Asp Leu Lys Asn Val Lys Ser Lys
   1 5 10 15
   Ile Gly Ser Thr Glu Asn Leu Lys His Gln Pro Gly Gly Gly Lys Val
   20 25 30
   Gln Ile Val Glu Lys Pro Val Asp Leu Ser Lys Val Thr Ser Lys Cys
   35 40 45
   Gly Ser Leu Gly Asn Ile His His Lys Pro Gly Gly Gly Gln Val Glu
   50 55 60
   Val Lys Ser Glu Lys Leu Asp Phe Lys Asp Arg Val Gln Ser Lys Ile
   65 70 75 80
   Gly Ser Leu Asp Asn Ile Thr His Val Pro Gly Gly Gly Asn
   85 90
<210> 14
   <211> 9
   <212> PRT
   <213> Homo sapiens
<400> 14
   Val Asp Leu Ser Lys Val Thr Ser Lys
   1 5
<210> 15
   <211> 18
   <212> PRT
   <213> Homo sapiens
<400> 15
   Val Thr Ser Lys Cys Gly Ser Leu Gly Asn Ile His His Lys Pro Gly
   1 5 10 15
   Gly Gly
<210> 16
   <211> 8
   <212> PRT
   <213> Homo sapiens
<400> 16
   Gly Gln Val Glu Val Lys Ser Glu
   1 5
<210> 17
   <211> 9
   <212> PRT
   <213> Homo sapiens
<400> 17
   Val Asp Leu Ser Lys Val Thr Ser Lys
   1 5
<210> 18
   <211> 18
   <212> PRT
   <213> Homo sapiens
<400> 18
   Val Thr Ser Lys Cys Gly Ser Leu Gly Asn Ile His His Lys Pro Gly Gly
   1 5 10 15
   Gly
<210> 19
   <211> 8
   <212> PRT
   <213> Homo sapiens
<400> 19
   Gly Gln Val Glu Val Lys Ser Glu
   1 5
<210> 20
   <211> 7
   <212> PRT
   <213> Homo sapiens
<400> 1
<210> 21
   <211> 6
   <212> PRT
   <213> Homo sapiens
<400> 2
<210> 22
   <211> 15
   <212> PRT
   <213> Homo sapiens
<400> 3
<210> 23
   <211> 15
   <212> PRT
   <213> Homo sapiens
<400> 4
<210> 24
   <211> 15
   <212> PRT
   <213> Homo sapiens
<400> 5
<210> 25
   <211> 15
   <212> PRT
   <213> Homo sapiens
<400> 6
<210> 26
   <211> 15
   <212> PRT
   <213> Homo sapiens
<400> 7
<210> 27
   <211> 15
   <212> PRT
   <213> Homo sapiens
<400> 8
<210> 28
   <211> 15
   <212> PRT
   <213> Homo sapiens
<400> 9
<210> 29
   <211> 15
   <212> PRT
   <213> Homo sapiens
<400> 10
<210> 30
   <211> 15
   <212> PRT
   <213> Homo sapiens
<400> 11
<210> 31
   <211> 15
   <212> PRT
   <213> Homo sapiens
<400> 12
<210> 32
   <211> 15
   <212> PRT
   <213> Homo sapiens
<400> 13
<210> 33
   <211> 15
   <212> PRT
   <213> Homo sapiens
<400> 14
<210> 34
   <211> 15
   <212> PRT
   <213> Homo sapiens
<400> 15
<210> 35
   <211> 15
   <212> PRT
   <213> Homo sapiens
<400> 16
<210> 36
   <211> 15
   <212> PRT
   <213> Homo sapiens
<400> 17
<210> 37
   <211> 15
   <212> PRT
   <213> Homo sapiens
<400> 18
<210> 38
   <211> 15
   <212> PRT
   <213> Homo sapiens
<400> 19
<210> 39
   <211> 15
   <212> PRT
   <213> Homo sapiens
<400> 20
<210> 40
   <211> 15
   <212> PRT
   <213> Homo sapiens
<400> 21
<210> 41
   <211> 15
   <212> PRT
   <213> Homo sapiens
<400> 22
<210> 42
   <211> 15
   <212> PRT
   <213> Homo sapiens
<400> 23
<210> 43
   <211> 15
   <212> PRT
   <213> Homo sapiens
<400> 24
<210> 44
   <211> 15
   <212> PRT
   <213> Homo sapiens
<400> 25
<210> 45
   <211> 15
   <212> PRT
   <213> Homo sapiens
<400> 26
<210> 46
   <211> 15
   <212> PRT
   <213> Homo sapiens
<400> 27
<210> 47
   <211> 15
   <212> PRT
   <213> Homo sapiens
<400> 28
<210> 48
   <211> 15
   <212> PRT
   <213> Homo sapiens
<400> 29
<210> 49
   <211> 15
   <212> PRT
   <213> Homo sapiens
<400> 30
<210> 50
   <211> 15
   <212> PRT
   <213> Homo sapiens
<400> 31
<210> 51
   <211> 15
   <212> PRT
   <213> Homo sapiens
<400> 32
<210> 52
   <211> 21
   <212> PRT
   <213> Homo sapiens
<400> 1
<210> 53
   <211> 4
   <212> PRT
   <213> Homo sapiens
<400> 2
<210> 54
   <211> 26
   <212> PRT
   <213> Homo sapiens
<400> 3
<210> 55
   <211> 9
   <212> PRT
   <213> Homo sapiens
<400> 4
<210> 56
   <211> 12
   <212> PRT
   <213> Homo sapiens
<400> 5
<210> 57
   <211> 19
   <212> PRT
   <213> Homo sapiens
<400> 6
<210> 58
   <211> 24
   <212> PRT
   <213> Homo sapiens
<400> 7
<210> 59
   <211> 30
   <212> PRT
   <213> Homo sapiens
<400> 8

## Claims

1. A method for identifying and obtaining an inhibitor, capable of modifying the paired-helical filament (PHF) formation comprising the steps of
(a) incubating a peptide consisting of a specific tau derived peptide as shown in any one of SEQ ID NOs 6, 7, 8 and 9 with a compound to be screened under conditions which permit assembly of said tau-derived peptides into nucleation sites for PHF assembly and/or into aggregation products; and
(b) detecting the presence, decrease, or absence of nucleation sites for PHF assembly and/or the presence, decrease or absence of aggregation products wherein said absence and/or decrease is indicative for putative inhibitors for PHF formation.

2. The method of claim 1 wherein in step (a) said incubation is carried out in the presence of assembly-competent tau protein(s) and/or assembly-competent tau protein fragments and wherein in step (b) the presence, decrease and/or absence of PHF or tau filaments is detected.

3. A method for identifying and obtaining an inhibitor of PHF formation comprising the steps of:
(a) contacting a peptide consisting of a specific tau-derived peptide as shown in any one of SEQ ID NOs. 6, 7, 8 or 9 with the first molecule known to bind to said peptide to form a first complex of said peptide and said first molecule;
(b) contacting said first complex with a compound to be screened; and
(c) measuring whether said compound displaces said first molecule from said first complex.

4. The method of claim 3 wherein said measuring step (c) comprises the measurement of a formation of a second complex of said tau-derived peptide and said compound.

5. The method of claim 3 or 4 wherein said measuring step (c) comprises the measurement of the amount of said first molecule that is not bound to said tau-derived peptide.

6. A method for the preparation of a pharmaceutical composition consisting of the steps of a method of any one of claims 1 to 5; and synthesizing and/or formulating the compounds identified, obtained and/or screened in step (b) or derivative thereof in a pharmaceutical acceptable form.

7. The method of any one of claims 1 to 6 wherein said peptide consisting of a tau-derived peptide as shown in any one of SEQ ID Nos. 6, 7, 8 and 9 is bound to a solid phase.

8. The method of any one of claims 1 to 7, wherein said incubation step is carried out in the presence of (poly)anions.

9. A method for detecting and/or measuring PHF formation comprising the steps of:
(a) incubating a peptide consisting of a specific tau derived peptide as shown in any one of SEQ ID NOs. 6, 7, 8 or 9 with tau-protein(s) and/or fragments thereof under conditions which permit assembly of tau-proteins and/or fragments thereof into PHFs; and
(b) detecting the presence, absence, decrease or increase of PHFs and/or nucleation sites of PHF assembly.

10. The method of any one of claims 1 to 9, wherein said detection or measuring step is carried out by microscopical or spectroscopical means.

11. The method of claim 10 wherein said microscopical means comprises electron microscopy.

12. The method of claim 10 wherein said spectroscopical means comprises circular dichroism spectroscopy or fluorescence spectroscopy, or infrared spectroscopy.

13. The method of claim 12, wherein said fluorescence spectroscopy comprises a thioflavin S or T assay or a tyrosine self-fluorescence assay.

14. A kit comprising:
(a) a tau derived peptide consisting of a peptide as shown in any one of SEQ ID NOs 6, 7, 8 or 9 and/or a nucleic acid molecule encoding said peptide or a solid support as defined in claim 7; and
(b) tau-protein(s) and/or fragments thereof as defined in claims 2 and/or 9, adopted for carrying out the method of claims 1 to 13, optionally further containing of a standard and/or suitable means for detection.

15. Use of the kit of claim 14 for carrying out the method of any one of claims 1 to 13.

16. Use of a peptide consisting of a specific tau derived peptide as shown in SEQ ID Nos. 6, 7, 8 or 9 or a nucleic acid molecule encoding a specific tau derived peptide as shown in SEQ ID NOS. 6, 7, 8 or 9 for carrying out the method of any one of claims 1 to 13.

17. Use of a peptide consisting of a specific tau derived peptide as shown in SEQ ID Nos. 6, 7, 8 or 9 or a nucleic acid molecule encoding a specific tau derived peptide as defined in SEQ ID NOS. 6, 7, 8 or 9 for screening and/or for the identification of an inhibitor, capable of modifying PHF formation.

18. The use of claims 16 or 17 or the method of any one of claims 1 to 6 and 9 wherein said peptide consisting of a specific tau derived peptide as shown in SEQ ID NOS. 6, 7, 8 or 9 is expressed in a non-human transgenic animal, a tissue culture and/or a cell culture system.

19. The use of any one of claims 16 to 18 or the method of any one of claims 1 to 6 and 18 wherein said peptide consisting of a specific tau derived peptide as shown in SEQ ID NOS 6, 7, 8 or 9 further comprises in the hexapeptide as defined in SEQ ID NOS 7 or 8 at least one mutation/modification.

20. The use or the method of claim 19, wherein said mutation and/or modification comprises the replacement of at least one amino acid with high intrinsic β-sheet propensity with at least one amino acid with low or no intrinsic β-sheet propensity.

## Patentansprüche

1. Verfahren zur Identifizierung und Erlangung eines Inhibitors, der in der Lage ist, die Bildung der paarigen helikalen Filamente (PHF) zu modifizieren, umfassend die Schritte:
(a) Inkubation eines Peptids bestehend aus einem spezifischen, von Tau abgeleiteten Peptid, wie dargestellt in einer der SEQ ID NO: 6, 7, 8 und 9, mit einer zu prüfenden Verbindung unter Bedingungen, welche eine Assemblierung der von Tau abgeleiteten Peptide in Nukleationsstellen, zur PHF-Assemblierung und/oder in Aggregationsprodukte zulassen; und
(b) Nachweis der Anwesenheit, Abnahme oder Abwesenheit von Nukleationsstellen zur PHF-Assemblierung und/oder der Anwesenheit, Abnahme oder Abwesenheit von Aggregationsprodukten, wobei die Abwesenheit und/oder Abnahme auf putative Inhibitoren der PHF-Bildung hinweist.

2. Verfahren nach Anspruch 1, wobei in Schritt (a) die Inkubation in Gegenwart von assemblierungsfähigem/n Tau-Protein(en) und/oder assemblierungsfähigen Tau-Proteinfragmenten durchgeführt wird und wobei in Schritt (b) die Anwesenheit, Abnahme und/oder Abwesenheit von PHF oder Tau-Filamenten nachgewiesen wird.

3. Verfahren zur Identifizierung und Erlangung eines Inhibitors der PHF-Bildung, umfassend die Schritte:
(a) Inkontaktbringen eines Peptids bestehend aus einem spezifischen, von Tau abgeleiteten Peptid, wie dargestellt in einer der SEQ ID NO: 6, 7, 8 oder 9, mit dem ersten Molekül, von dem bekannt ist, dass es an das Peptid bindet, um einen ersten Komplex aus Peptid und erstem Molekül zu bilden;
(b) Inkontaktbringen des ersten Komplexes mit einer zu prüfenden Verbindung; und
(c) Messen, ob die Verbindung das erste Molekül von dem ersten Komplex verdrängt.

4. Verfahren nach Anspruch 3, wobei der Messschritt (c) die Messung der Bildung eines zweiten Komplexes des von Tau abgeleiteten Peptids und der Verbindung umfasst.

5. Verfahren nach Anspruch 3 oder 4, wobei der Messschritt (c) die Messung der Menge des ersten Moleküls umfasst, die nicht an das von Tau abgeleitete Peptid gebunden ist.

6. Verfahren zur Herstellung eines Arzneimittels bestehend aus den Schritten eines Verfahrens nach einem der Ansprüche 1 bis 5; und Synthetisieren und/oder Formulieren der Verbindungen, die in Schritt (b) identifiziert, erhalten und/oder geprüft wurden oder ein Derivat davon in einer pharmazeutisch verträglichen Form.

7. Verfahren nach einem der Ansprüche 1 bis 6, wobei das Peptid bestehend aus einem von Tau abgeleiteten Peptid, wie dargestellt in einer der SEQ ID NO: 6, 7, 8 und 9, an eine Festphase gebunden ist.

8. Verfahren nach einem der Ansprüche 1 bis 7, wobei der Inkubationsschritt in Gegenwart von (Poly)Anionen durchgeführt wird.

9. Verfahren zum Nachweis und/oder Messen der PHF-Bildung, umfassend die Schritte:
(a) Inkubation eines Peptids bestehend aus einem spezifischen, von Tau abgeleiteten Peptid, wie dargestellt in einer der SEQ ID NO: 6, 7, 8 oder 9, mit Tau-Protein(en) und/oder Fragmenten davon unter Bedingungen, welche eine Assemblierung der Tau-Proteine und/oder Fragmente davon zu PHFs zulässt; und
(b) Nachweis der Anwesenheit, Abwesenheit, Abnahme oder Zunahme von PHFs und/oder Nukleationsstellen zur PHF-Assemblierung.

10. Verfahren nach einem der Ansprüche 1 bis 9, wobei der Nachweis- oder Messschritt mit mikroskopischen oder spektroskopischen Mitteln ausgeführt wird.

11. Verfahren nach Anspruch 10, wobei das mikroskopische Mittel Elektronenmikroskopie umfasst.

12. Verfahren nach Anspruch 10, wobei das spektroskopische Mittel Circular-Dichroismus-Spektroskopie oder Fluoreszenz-Spektroskopie oder Infrarot-Spektroskopie umfasst.

13. Verfahren nach Anspruch 12, wobei die Fluoreszenz-Spektroskopie einen Thioflavin-S- oder -T-Test oder einen Tyrosin-Eigenfluoreszenz-Test umfasst.

14. Kit umfassend:
(a) ein von Tau abgeleitetes Peptid, bestehend aus einem Peptid, wie dargestellt in einer der SEQ ID NO: 6, 7, 8 oder 9, und/oder ein Nucleinsäuremolekül codierend für das Peptid oder einen festen Träger wie definiert in Anspruch 7; und
(b) Tau Protein(e) und/oder Fragmente davon, wie definiert in Ansprüchen 2 und/oder 9,
angepasst zur Ausführung des Verfahrens nach Ansprüchen 1 bis 13, gegebenenfalls zusätzlich enthaltend einen Standard und/oder geeignete Mittel zum Nachweis.

15. Verwendung des Kits nach Anspruch 14 zur Ausführung des Verfahrens nach einem der Ansprüche 1 bis 13.

16. Verwendung eines Peptids bestehend aus einem spezifischen, von Tau abgeleiteten Peptid, wie dargestellt in SEQ ID NO: 6, 7, 8 oder 9, oder eines Nucleinsäuremoleküls codierend ein spezifisches, von Tau abgeleitetes Peptid, wie dargestellt in SEQ ID NO: 6, 7, 8 oder 9, zur Ausführung des Verfahrens nach einem der Ansprüche 1 bis 13.

17. Verwendung eines Peptids bestehend aus einem spezifischen, von Tau abgeleiteten Peptid, wie dargestellt in SEQ ID NO: 6, 7, 8 oder 9, oder eines Nucleinsäuremoleküls codierend ein spezifisches, von Tau abgeleitetes Peptid, wie dargestellt in SEQ ID NO: 6, 7, 8 oder 9, zum Screenen für und/oder Identifizieren eines Inhibitors, der in der Lage ist, die PHF-Bildung zu modifizieren.

18. Verwendung nach Ansprüchen 16 oder 17 oder Verfahren nach einem der Ansprüche 1 bis 6 und 9, wobei das Peptid bestehend aus einem spezifischen, von Tau abgeleiteten Peptid, wie dargestellt in SEQ ID NO: 6, 7, 8 oder 9, in einem nicht-menschlichen, transgenen Tier, einer Gewebekultur, und/oder einem Zellkultursystem exprimiert wird.

19. Verwendung nach einem der Ansprüche 16 bis 18 oder Verfahren nach einem der Ansprüche 1 bis 6 und 18, wobei das Peptid, bestehend aus einem spezifischen, von Tau abgeleiteten Peptid, wie dargestellt in SEQ ID NO: 6, 7, 8 oder 9, außerdem in dem Hexapeptid wie definiert in SEQ ID NO: 7 oder 8 mindestens eine Mutation/Modifikation umfasst.

20. Verwendung oder Verfahren nach Anspruch 19, wobei die Mutation und/oder Modifikation den Austausch von mindestens einer Aminosäure, mit hoher intrinsischer Neigung zu β-Faltblattstruktur mit mindestens einer Aminosäure mit geringer oder keiner intrinsischer Neigung zu β-Faltblattstruktur umfasst.

## Revendications

1. Méthode d'identification et d'obtention d'un inhibiteur, capable de modifier la formation du filament en paire hélicoïdale (PHF) comprenant les étapes
(a) d'incubation d'un peptide consistant en un peptide spécifique dérivé de tau tel que montré dans l'une quelconque des SEQ ID Nos 6, 7, 8 et 9 avec un composé à cribler dans des conditions qui permettent l'assemblage desdits peptides dérivés de tau dans des sites de nucléation pour l'assemblage du PHF et/ou dans des produits d'agrégation ; et
(b) de détection de la présence, de la diminution, ou de l'absence de sites de nucléation pour l'assemblage du PHF et/ou de la présence, de la diminution, ou de l'absence de produits d'agrégation dans laquelle ladite absence et/ou diminution est indicative d'inhibiteurs présumés de la formation du PHF.

2. Méthode selon la revendication 1 dans laquelle dans l'étape (a) ladite incubation est réalisée en présence de protéine(s) tau capable(s) d'assemblage et/ou de fragments de protéine tau capables d'assemblage et dans laquelle lors de l'étape (b) la présence, la diminution et/ou l'absence de filaments PHF ou tau est détectée.

3. Méthode d'identification et d'obtention d'un inhibiteur de la formation du PHF comprenant les étapes :
(a) de mise en contact d'un peptide consistant en un peptide spécifique dérivé de tau tel que montré dans l'une quelconque des SEQ ID Nos 6, 7, 8 et 9 avec la première molécule connue pour lier ledit peptide de façon à former un premier complexe dudit peptide et de ladite première molécule ;
(b) de mise en contact dudit premier complexe avec un composé à cribler ; et
(c) de mesure du déplacement éventuel par ledit composé de ladite première molécule dudit premier complexe.

4. Méthode selon la revendication 3 dans laquelle ladite étape de mesure (c) comprend la mesure d'une formation d'un second complexe dudit peptide dérivé de tau et dudit composé.

5. Méthode selon la revendication 3 ou 4 dans laquelle ladite étape de mesure (c) comprend la mesure de la quantité de ladite première molécule qui n'est pas liée audit peptide dérivé de tau.

6. Méthode de préparation d'une composition pharmaceutique consistant en les étapes d'une méthode selon l'une quelconque des revendications 1 à 5 ; et en la synthèse et/ou la formulation des composés identifiés, obtenus et/ou criblés lors de l'étape (b) ou de dérivés de ceux-ci dans une forme acceptable sur le plan pharmaceutique.

7. Méthode selon l'une quelconque des revendications 1 à 6 dans laquelle ledit peptide consistant en un peptide dérivé de tau tel que montré dans l'une quelconque des SEQ ID Nos 6, 7, 8 et 9 est lié à une phase solide.

8. Méthode selon l'une quelconque des revendications 1 à 7, dans laquelle ladite étape d'incubation est réalisée en présence de (poly)anions.

9. Méthode de détection et/ou mesure de la formation du PHF comprenant les étapes :
(a) d'incubation d'un peptide consistant en un peptide spécifique dérivé de tau tel que montré dans l'une quelconque des SEQ ID NOs. 6, 7, 8 ou 9 avec une ou des protéines tau et/ou des fragments de celles-ci dans des conditions permettant l'assemblage de protéines-tau et/ou fragments de celles-ci en PHFs ; et
(b) de détection de la présence, de l'absence, de la diminution ou de l'augmentation du PHFs et/ou de sites de nucléation d'assemblage du PHF.

10. Méthode selon l'une quelconque des revendications 1 à 9, dans laquelle ladite étape de détection ou de mesure est réalisée par des moyens microscopique ou spectroscopique.

11. Méthode selon la revendication 10 dans laquelle ledit moyen microscopique comprend la microscopie électronique.

12. Méthode selon la revendication 10 dans laquelle ledit moyen spectroscopique comprend la spectroscopie par dichroïsme circulaire ou la spectroscopie par fluorescence, ou la spectroscopie infrarouge.

13. Méthode selon la revendication 12, dans laquelle ladite spectroscopie par fluorescence comprend un test de thioflavine S ou T ou un test de tyrosine par auto-fluorescence.

14. Kit comprenant :
(a) un peptide dérivé de tau consistant en un peptide tel que montré dans l'une quelconque des SEQ ID Nos 6, 7, 8 ou 9 et/ou une molécule d'acide nucléique codant ledit peptide ou un support solide tel que défini dans la revendication 7 ; et
(b) une ou des protéines tau et/ou fragments de celles-ci tels que définis dans les revendications 2 et/ou 9 adaptés pour réaliser la méthode selon les revendications 1 à 13, consistant éventuellement en outre en un standard et/ou des moyens adapté à la détection.

15. Utilisation du kit selon la revendication 14 pour réaliser la méthode de l'une quelconque des revendications 1 à 13.

16. Utilisation d'un peptide consistant en un peptide spécifique dérivé de tau tel que montré dans SEQ ID Nos. 6, 7, 8 ou 9 ou une molécule d'acide nucléique codant un peptide spécifique dérivé de tau tel que montré dans SEQ ID Nos. 6, 7, 8 ou 9 pour réaliser la méthode selon l'une quelconque des revendications 1 à 13.

17. Utilisation d'un peptide consistant en un peptide spécifique dérivé de tau tel que montré dans SEQ ID Nos. 6, 7, 8 ou 9 ou une molécule d'acide nucléique codant un peptide spécifique dérivé de tau tel que défini dans SEQ ID Nos. 6, 7, 8 ou 9 pour le criblage et/ou pour l'identification d'un inhibiteur, capable de modifier la formation du PHF.

18. Utilisation selon les revendications 16 ou 17 ou méthode selon l'une quelconque des revendications 1 à 6 et 9 dans laquelle ledit peptide consistant en un peptide spécifique dérivé de tau tel que montré dans SEQ ID Nos. 6, 7, 8 ou 9 est exprimé dans un animal transgénique non humain, une culture tissulaire et/ou un système de culture cellulaire.

19. Utilisation selon l'une quelconque des revendications 16 à 18 ou méthode selon l'une quelconque des revendications 1 à 6 et 18 dans laquelle ledit peptide consistant en un peptide spécifique dérivé de tau tel que montré dans des SEQ ID Nos 6, 7, 8 ou 9 comprend en outre dans l'hexapeptide tel que défini dans SEQ ID Nos. 7 ou 8 au moins une mutation/modification.

20. Utilisation ou méthode selon la revendication 19, dans laquelle ladite mutation et/ou modification comprend le remplacement d'au moins un acide aminé avec une forte propension intrinsèque au feuillet β par au moins un acide aminé avec une faible ou aucune propension intrinsèque au feuillet β.
